# EUROPÄISCHE PATENTSCHRIFT

(11) **EP 1 178 985 B1**
(45) Veröffentlichungstag und Bekanntmachung des Hinweises auf die Patenterteilung: **09.04.2003**
(21) Anmeldenummer: 00927120.6
(22) Anmeldetag: 02.05.2000
(51) Int. Cl.: C07D 409/12, C07D 333/54, C07D 333/62, C07D 333/72, C07D 333/78, C07D 333/80, A01N 43/68

(54) **HERBIZIDE SUBSTITUIERTE THIENOCYCLOALK(EN)YLAMINO-1,3,5-TRIAZINE**
SUBSTITUTED THIENOCYCLOALK(EN)YLAMINO-1,3,5-TRIAZINE
THIENOCYCLOALK(EN)YLAMINO-1,3,5-TRIAZINES SUBSTITUEES

(30) Priorität: 12.05.1999 DE 19921883
(43) Veröffentlichungstag der Anmeldung: 13.02.2002
(73) Patentinhaber: Bayer CropScience AG, 40789 Monheim (DE)
(72) Erfinder: KATHER, Kristian, D-40789 Monheim (DE); LEHR, Stefan, D-40764 Langenfeld (DE); RIEBEL, Hans-Jochem, D-56242 Selters (DE); VOIGT, Katharina, D-40789 Monheim (DE); DREWES, Mark, Wilhelm, D-40764 Langenfeld (DE); FEUCHT, Dieter, D-40789 Monheim (DE); PONTZEN, Rolf, D-42799 Leichlingen (DE); WETCHOLOWSKY, Ingo, CEP-13280-000 Vinhedo, SP (BR)
(86) Internationale Anmeldenummer: EP0003928
(87) Internationale Veröffentlichungsnummer: WO00069854

(56) Entgegenhaltungen:
- EP-A- 0 283 522
- DE-A- 19 744 232
- US-A- 4 740 230

## Beschreibung

Die Erfindung betrifft neue substituierte Thienocycloalk(en)ylamino-1,3,5-triazine, Verfahren zu ihrer Herstellung einschließlich der neuen Zwischenprodukte und ihre Verwendung als Herbizide.

Eine Reihe von substituierten Thienylalkylamino-1,3,5-triazinen ist bereits aus der (Patent-)Literatur bekannt (vgl. WO-A-98/15537, WO-A-98/15539, DE-A-1974 4232). Diese Verbindungen haben jedoch bisher keine besondere Bedeutung erlangt. Weiterhin sind Triazine bekannt, bei denen ein bicyclisches aromatisches System aus einem 5- und einem 6-Ring über den 5-Ring und eine (gegebenenfalls substituierte) Methyleneinheit an einen Aminosubstituenten der Triazinsystems gebunden ist (vgl. US-A 4740230, EP-A-0 283 522). Substituierte Thienocycloalk(en)ylamino-1,3,5-triazine sind bisher überhaupt nicht bekannt geworden.

Es wurden nun die neuen substituierten Thienocycloalk(en)ylamino-1,3,5-triazine der allgemeinen Formel (I) 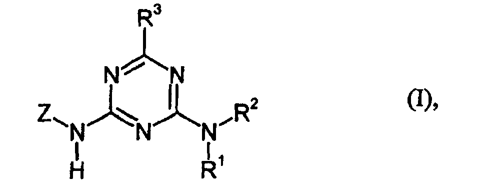 in welcher
- R¹: für Wasserstoff oder für gegebenenfalls substituiertes Alkyl steht,
- R²: für Wasserstoff, für Formyl oder für jeweils gegebenenfalls substituiertes Alkyl, Alkylcarbonyl, Alkoxycarbonyl oder Alkylaminocarbonyl steht,
oder die Gruppierung N(R¹R²) auch für Dialkylaminoalkylidenamino steht,
- R³: für Wasserstoff, für Halogen, für gegebenenfalls substituiertes Alkyl, fiir jeweils gegebenenfalls substituiertes Alkylcarbonyl, Alkoxycarbonyl, Alkoxy, Alkylthio, Alkylsulfinyl oder Alkylsulfonyl, für jeweils gegebenenfalls substituiertes Alkenyl oder Alkinyl, oder für gegebenenfalls substituiertes Cycloalkyl steht, und
- Z: für eine der nachstehenden Thienocycloalk(en)yl-Gruppierungen steht 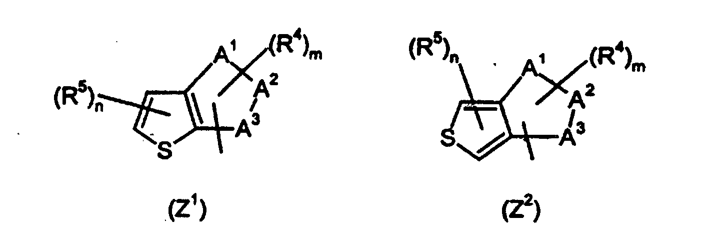 worin
m für die Zahlen 0, 1, 2, 3 oder 4 steht,
n für die Zahlen 0, 1 oder 2 steht,
A¹ für O (Sauerstoff), S (Schwefel), -CO-, -CS- oder Alkandiyl (Alkylen) steht,
A² für O (Sauerstoff), S (Schwefel), -CO-, -CS- oder Alkandiyl (Alkylen) steht,
A³ für O (Sauerstoff), S (Schwefel), -CO-, -CS- oder Alkandiyl (Alkylen) steht,
- mit der Maßgabe, daß von den Gruppierungen A¹, A², A³ wenigstens eine für Alkandiyl steht und nicht zwei benachbarte Gruppen gleichzeitig für S oder O stehen -

R⁴ für Amino, Cyano, Carbamoyl, Thiocarbamoyl, Formyl, Halogen, oder für jeweils gegebenenfalls substituiertes Alkyl, Alkylcarbonyl, Alkoxy, Alkoxycarbonyl, Alkylthio, Alkylsulfinyl, Alkylsulfonyl, Alkylamino, Dialkylamino, Alkylcarbonylamino, Alkoxycarbonylamino, Alkylsulfonylamino, Alkenyl, Alkinyl, Alkenylcarbonyl, Alkinylcarbonyl, Aryl, Arylcarbonyl oder Arylalkyl steht, und
R⁵ für Nitro, Amino, Cyano, Carbamoyl, Thiocarbamoyl, Formyl, Halogen, oder für jeweils gegebenenfalls substituiertes Alkyl, Alkylcarbonyl, Alkoxy, Alkoxycarbonyl, Alkylthio, Alkylsulfinyl, Alkylsulfonyl, Alkylamino, Dialkylamino, Alkylcarbonylamino, Alkoxycarbonylamino, Alkylsulfonylamino, Alkenyl, Alkinyl, Alkenylcarbonyl, Alkinylcarbonyl, Aryl, Arylcarbonyl oder Arylalkyl steht, gefunden.

Gesättigte oder ungesättigte Kohlenwasserstoffgruppierungen, wie Alkyl, Alkandiyl, Alkenyl oder Alkinyl, sind - auch in Verknüpfungen mit Heteroatomen, wie in Alkoxy - soweit möglich jeweils geradkettig oder verzweigt.

Gegebenenfalls substituierte Reste können einfach oder mehrfach substituiert sein, wobei bei Mehrfächsubstituition die Substituenten gleich oder verschieden sein können.

Bevorzugte Substituenten bzw. Bereiche der in den oben und nachstehend aufgeführten Formeln vorhandenen Reste werden im folgenden definiert.
- m: steht bevorzugt für die Zahlen 0, 1 oder 2.
- A¹: steht bevorzugt für O (Sauerstoff), S (Schwefel), -CO-, -CS- oder Alkandiyl (Alkylen) mit 1 bis 3 Kohlenstoffatomen.
- A²: steht bevorzugt für O (Sauerstoff), S (Schwefel), -CO-, -CS- oder Alkandiyl (Alkylen) mit 1 bis 3 Kohlenstoffatomen.
- A³: steht bevorzugt für O (Sauerstoff), S (Schwefel), -CO-, -CS- oder Alkandiyl (Alkylen) mit 1 bis 3 Kohlenstoffatomen.
Von den Gruppierungen A¹, A², A³ steht bei den bevorzugten Verbindungen wenigstens eine für Alkandiyl mit 1 bis 3 Kohlenstoffatomen, und zwei benachbarte Gruppen stehen nicht gleichzeitig für S oder O.
- R¹: steht bevorzugt für Wasserstoff oder für gegebenenfalls durch Cyano, Halogen oder C₁-C₄-Alkoxy substituiertes Alkyl mit 1 bis 6 Kohlenstoffatomen.
- R²: steht bevorzugt für Wasserstoff, fiir Formyl oder für jeweils gegebenenfalls durch Cyano, Halogen oder C₁-C₄-Alkoxy substituiertes Alkyl, Alkylcarbonyl, Alkoxycarbonyl oder Alkylaminocarbonyl mit jeweils 1 bis 6 Kohlenstoffatomen in den Alkylgruppen.
Die Gruppierung N(R¹R²) steht bevorzugt auch für Dialkylaminoalkylidenamino mit jeweils bis zu 4 Kohlenstoffatomen in den Alkylgruppen bzw. Alkylidengruppen.
- R³: steht bevorzugt für Wasserstoff, für Halogen, für gegebenenfalls durch Cyano, Halogen, Hydroxy, C₁-C₄-Alkoxy oder C₁-C₄-Alkylthio substituiertes Alkyl mit 1 bis 6 Kohlenstoffatomen, fiir jeweils gegebenenfalls durch Cyano, Halogen oder C₁-C₄-Alkoxy substituiertes Alkylcarbonyl, Alkoxycarbonyl, Alkoxy, Alkylthio, Alkylsulfinyl oder Alkylsulfonyl mit jeweils 1 bis 6 Kohlenstoffatomen in den Alkylgruppen, für jeweils gegebenenfalls durch Halogen substituiertes Alkenyl oder Alkinyl mit jeweils 2 bis 6 Kohlenstoffatomen, oder für gegebenenfalls durch Cyano, Halogen oder C₁-C₄-Alkyl substituiertes Cycloalkyl mit 3 bis 6 Kohlenstoffatomen.
- R⁴: steht bevorzugt für Amino, Cyano, Carbamoyl, Thiocarbamoyl, Formyl, Halogen, für jeweils gegebenenfalls durch Cyano, Halogen oder C₁-C₄-Alkoxy substituiertes Alkyl, Alkylcarbonyl, Alkoxy, Alkoxycarbonyl, Alkylthio, Alkylsulfinyl, Alkylsulfonyl, Alkylamino, Dialkylamino, Alkylcarbonylamino, Alkoxycarbonylamino oder Alkylsulfonylamino mit jeweils 1 bis 6 Kohlenstoffatomen in den Alkylgruppen, für jeweils gegebenenfalls durch Cyano oder Halogen substituiertes Alkenyl, Alkinyl, Alkenylcarbonyl oder Alkinylcarbonyl mit jeweils 2 bis 6 Kohlenstoffatomen in den Alkenyl- bzw. Alkinylgruppen, oder für jeweils gegebenenfalls durch Nitro, Cyano, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl, Arylcarbonyl oder Arylalkyl mit jeweils 6 oder 10 Kohlenstoffatomen in der Arylgruppe und gegebenenfalls 1 bis 4 Kohlenstoffatomen im Alkylteil.
- R⁵: steht bevorzugt für Nitro, Amino, Cyano, Carbamoyl, Thiocarbamoyl, Formyl, Halogen, fiir jeweils gegebenenfalls durch Cyano, Halogen oder C₁-C₄-Alkoxy substituiertes Alkyl, Alkylcarbonyl, Alkoxy, Alkoxycarbonyl, Alkylthio, Alkylsulfinyl, Alkylsulfonyl, Alkylamino, Dialkylamino, Alkylcarbonylamino, Alkoxycarbonylamino oder Alkylsulfonylamino mit jeweils 1 bis 6 Kohlenstoffatomen in den Alkylgruppen, für jeweils gegebenenfalls durch Cyano oder Halogen substituiertes Alkenyl, Alkinyl, Alkenylcarbonyl oder Alkinylcarbonyl mit jeweils 2 bis 6 Kohlenstoffatomen in den Alkenyl- bzw. Alkinylgruppen, oder für jeweils gegebenenfalls durch Nitro, Cyano, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl, Arykarbonyl oder Arylalkyl mit jeweils 6 oder 10 Kohlenstoffatomen in der Arylgruppe und gegebenenfalls 1 bis 4 Kohlenstoffatomen im Alkylteil.
- A¹: steht besonders bevorzugt für O (Sauerstoff), S (Schwefel), -CO-, -CS-, Methylen, Dimethylen oder Trimethylen.
- A²: steht besonders bevorzugt für O (Sauerstoff), S (Schwefel), -CO-, -CS-, Methylen, Dimethylen oder Trimethylen.
- A³: steht besonders bevorzugt für O (Sauerstoff), S (Schwefel), -CO-, -CS-, Methylen, Dimethylen oder Trimethylen.
Von den Gruppierungen A¹, A², A³ steht bei den bevorzugten Verbindungen wenigstens eine für Methylen, Dimethylen oder Trimethylen, und zwei benachbarte Gruppen stehen nicht gleichzeitig für S oder O.
- R¹: steht besonders bevorzugt für Wasserstoff oder für jeweils gegebenenfalls durch Cyano, Fluor, Chlor, Methoxy oder Ethoxy substituiertes Methyl, Ethyl, n- oder i-Propyl.
- R²: steht besonders bevorzugt für Wasserstoff, für Formyl oder für jeweils gegebenenfalls durch Cyano, Fluor, Chlor, Methoxy oder Ethoxy substituiertes Methyl, Ethyl, n- oder i-Propyl, Acetyl, Propionyl, n- oder i-Butyroyl, Methoxycarbonyl, Ethoxycarbonyl, n- oder i-Propoxycaibonyl, Methylanunocarbonyl, Ethylaminocarbonyl, n- oder i-Propylaminocarbonyl.
Die Gruppierung N(R¹R²) steht besonders bevorzugt auch für Dimethylaminomethylenamino oder Diethylaminomethylenamino.
- R³: steht besonders bevorzugt für Wasserstoff, für Fluor, Chlor, Brom, für jeweils gegebenenfalls durch Cyano, Fluor, Chlor, Brom, Hydroxy, Methoxy, Ethoxy, n- oder i-Propoxy, Methylthio, Ethylthio, n- oder i-Propylthio substituiertes Methyl, Ethyl, n- oder i-Propyl, n-, i- oder s-Butyl, für jeweils gegebenenfalls durch Cyano, Fluor, Chlor, Methoxy oder Ethoxy substituiertes Acetyl, Propionyl, n- oder i-Butyroyl, Methoxycarbonyl, Ethoxycarbonyl, n-oder i-Propoxycarbonyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, für jeweils gegebenenfalls durch Fluor, Chlor oder Brom substituiertes Ethenyl, Propenyl, Butenyl, Ethinyl, Propinyl oder Butinyl, oder für jeweils gegebenenfalls durch Cyano, Fluor, Chlor, Methyl oder Ethyl substituiertes Cyclopropyl, Cyclobutyl, Cyclopentyl oder Cyclohexyl.
- R⁴: steht besonders bevorzugt für Amino, Cyano, Carbamoyl, Tbiocarbamoyl, Formyl, Fluor, Chlor, Brom, für jeweils gegebenenfalls durch Cyano, Fluor, Chlor, Methoxy oder Ethoxy substituiertes Methyl, Ethyl, n- oder i-Propyl, Acetyl, Propionyl, n- oder i-Butyroyl, Methoxy, Ethoxy, n- oder i-Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Methylanimo, Ethylamino, n- oder i-Propylamino, Dimethylamino, Diethylamino, Acetylamino, Propionylamino, n- oder i-Butyroylamino, Methoxycarbonylamino, Ethoxycarbonylamino, n- oder i-Propoxycarbonylamino, Methylsulfonylamino, Ethylsulfonylamino, n- oder i-Propylsulfonylamino, für jeweils gegebenenfalls durch Cyano, Fluor, Chlor oder Brom substituiertes Ethenyl, Propenyl, Butenyl, Ethinyl, Propinyl, Butinyl, Ethenylcarbonyl, Propenylcarbonyl, Butenylcarbonyl, Ethinylcarbonyl, Propinylcarbonyl oder Butinylcarbonyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methoxycarbonyl, Ethoxycarbonyl, n- oder i-Propoxycarbonyl substituiertes Phenyl, Benzoyl oder Benzyl.
- R⁵: steht besonders bevorzugt für Nitro, Amino, Cyano, Carbamoyl, Thiocarbamoyl, Formyl, Fluor, Chlor, Brom, fiir jeweils gegebenenfalls durch Cyano, Fluor, Chlor, Methoxy oder Ethoxy substituiertes Methyl, Ethyl, n-oder i-Propyl, Acetyl, Propionyl, n- oder i-Butyroyl, Methoxy, Ethoxy, n-oder i-Propoxy, Methoxycarbonyl, Ethoxycarbonyl, n- oder i-Propoxycarbonyl, , Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, Dimethylamino, Diethylamino, Acetylamino, Propionylamino, n- oder i-Butyroylamino, Methoxycarbonylamino, Ethoxycarbonylamino, n- oder i-Propoxycarbonylamino, Methylsulfonylamino, Ethylsulfonylamino, n- oder i-Propylsulfonylamino, für jeweils gegebenenfalls durch Cyano, Fluor, Chlor oder Brom substituiertes Ethenyl, Propenyl, Butenyl, Ethinyl, Propinyl, Butinyl, Ethenylcarbonyl, Propenylcarbonyl, Butenylcarbonyl, Ethinylcarbonyl, Propinylcarbonyl oder Butinylcarbonyl, oder für jeweils gegebenenfalls durch Nitro, Cyano, Fluor, Chlor, Brom, Methyl, Ethyl, n- oder i-Propyl, n-, i-, s-oder t-Butyl, Trifluormethyl, Methoxy, Ethoxy, n- oder i-Propoxy, Difluormethoxy, Trifluormethoxy, Methoxycarbonyl, Ethoxycarbonyl, n- oder i-Propoxycarbonyl substituiertes Phenyl, Benzoyl oder Benzyl.
- A¹: steht ganz besonders bevorzugt für Methylen oder Dimethylen.
- A²: steht ganz besonders bevorzugt für Methylen oder Dimethylen.
- A³: steht ganz besonders bevorzugt für Methylen oder Dimethylen.
- R¹: steht ganz besonders bevorzugt für Wasserstoff.
- R²: steht ganz besonders bevorzugt fiir Wasserstoff, für Formyl oder für jeweils gegebenenfalls durch Fluor, Chlor, Methoxy oder Ethoxy substituiertes Acetyl, Propionyl, n- oder i-Butyroyl, Methoxycarbonyl, Ethoxycarbonyl, n-oder i-Propoxycarbonyl.
Die Gruppierung N(R¹R²) steht ganz besonders bevorzugt auch für Dimethylaminomethylenamino.
- R³: steht ganz besonders bevorzugt für jeweils gegebenenfalls durch Fluor oder Chlor substituiertes Methyl, Ethyl, n- oder i-Propyl.
- R⁴: steht ganz besonders bevorzugt für Cyano, Fluor, Chlor, Brom, oder für jeweils gegebenenfalls durch Fluor oder Chlor substituiertes Methyl, Ethyl, Methoxy oder Ethoxy.
- R⁵: steht ganz besonders bevorzugt fiir Nitro, Cyano, Fluor, Chlor, Brom, oder für jeweils gegebenenfalls durch Fluor oder Chlor substituiertes Methyl, Ethyl, Methoxy oder Ethoxy.

In der allgemeinen Formel (I) steht Z am meisten bevorzugt für 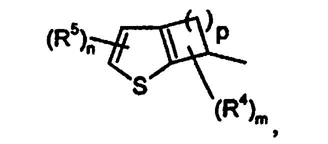 wobei
- p: für 2, 3 oder 4 steht und n, m, R⁴ und R⁵ wie oben angegeben definiert sind.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander; also auch zwischen den angegebenen bevorzugten Bereichen beliebig kombiniert werden.

Erfindungsgemäß bevorzugt sind diejenigen Verbindungen der Formel (I), bei welchen eine Kombination der vorstehend als bevorzugt aufgeführten Bedeutungen vorliegt.

Erfindungsgemäß besonders bevorzugt sind diejenigen Verbindungen der Formel (I), bei welchen eine Kombination der vorstehend als besonders bevorzugt aufgeführten Bedeutungen vorliegt.

Erfindungsgemäß ganz besonders bevorzugt sind diejenigen Verbindungen der Formel (I), bei welchen eine Kombination der vorstehend als ganz besonders bevorzugt aufgeführten Bedeutungen vorliegt.

Erfindungsgemäß am meisten bevorzugt sind diejenigen Verbindungen der Formel (I), bei welchen Z die als am meisten bevorzugt angegebene Bedeutung hat.

Gesättigte oder ungesättigte Kohlenwasserstoffreste, wie Alkyl, Alkandiyl oder Alkenyl, sind - auch in Verbindung mit Heteroatomen, wie in Alkoxy - soweit möglich jeweils geradkettig oder verzweigt.

Gegebenenfalls substituierte Reste können einfach oder mehrfach substituiert sein, wobei bei Mehrfachsubstitution die Substituenten gleich oder verschieden sein können.

Die erfindungsgemäßen Verbindungen der allgemeinen Formel (I) enthalten gegebenenfalls ein asymmetrisch substituiertes Kohlenstoffatom und können dann in verschiedenen enantiomeren (R- und S- konfigurierten Formen) bzw. diasteromeren Formen vorliegen. Die Erfindung betrifft sowohl die verschiedenen möglichen einzelnen enantiomeren bzw. stereoisomeren Formen der Verbindungen der allgemeinen Formel (I) wie auch die Gemische dieser isomeren Verbindungen.

Die neuen substituierten Thienocycloalk(en)ylamino-1,3,5-triazine der allgemeinen Formel (I) weisen interessante biologische Eigenschaften auf. Sie zeichnen sich insbesondere durch starke herbizide Wirksamkeit aus.

Man erhält die neuen substituierten Thienocycloalk(en)ylamino-1,3,5-triazine der allgemeinen Formel (I), wenn man Biguanide der allgemeinen Formel (II) 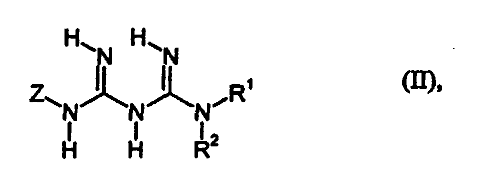 in welcher
R¹, R² und Z die oben angegebene Bedeutung haben,
   - und/oder Säureaddukte von Verbindungen der allgemeinen Formel (II) -
   mit Alkoxycarbonylverbindungen der allgemeinen Formel (III)

   R³-CO-OR⁴ (III)

   in welcher
   R³ die oben angegebene Bedeutung hat und
   R⁴ für Alkyl steht,
   gegebenenfalls in Gegenwart eines Reaktionshilfsmittels und gegebenenfalls in Gegenwart eines Verdünnungsmittels umsetzt und gegebenenfalls an den so erhaltenen Verbindungen der allgemeinen Formel (I) im Rahmen der Substituentendefinition weitere Umwandlungen nach üblichen Methoden durchführt.

Die Verbindungen der allgemeinen Formel (I) können nach üblichen Methoden in andere Verbindungen der allgemeinen Formel (I) gemäß obiger Substituentendefinition umgewandelt werden, beispielsweise durch Umsetzung von Verbindungen der Formel (I), in welcher R² für Wasserstoff steht, mit Acylierungsmitteln, wie z.B. Acetylchlorid, Acetanhydrid, Propionsäurechlorid, Propionsäureanhydrid, Chlorameisensäuremethylester oder Chlorameisensäureethylester (bei R² z.B. Einführung von COCH₃-, COC₂H₅-, COOCH₃-, COOC₂H₅-Gruppen für ein Wasserstoffatom).

Verwendet man beispielsweise 1-(4,5,6,7-Tetrahydro-benzo[b]thiophen-4-yl)-biguanid und Trifluoressigsäuremethylester als Ausgangsstoffe, so kann der Reaktionsablauf beim erfindungsgemäßen Verfahren durch das folgende Formelschema skizziert werden:

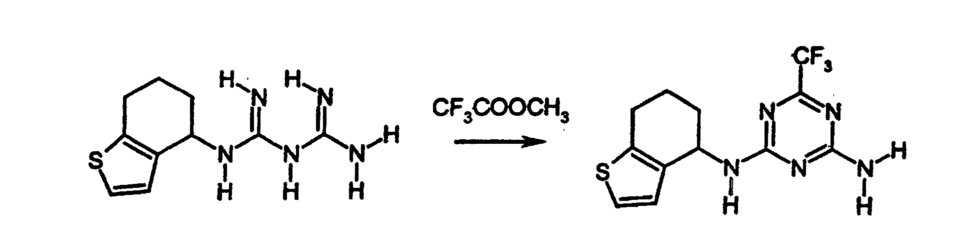

Die beim erfindungsgemäßen Verfahren zur Herstellung von Verbindungen der allgemeinen Formel (I) als Ausgangsstoffe zu verwendenden Biguanide sind durch die Formel (II) allgemein definiert. In der allgemeinen Formel (II) haben R¹, R² und Z vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für R¹, R² und Z angegeben worden sind.

Geeignete Säureaddukte von Verbindungen der Formel (II) sind deren Additionsprodukte mit Protonensäuren, wie z.B. mit Chlorwasserstoff (Hydrogenchlorid), Bromwasserstoff (Hydrogenbromid), Schwefelsäure, Methansulfonsäure, Benzolsulfonsäure und p-Toluolsulfonsäure.

Die Ausgangsstoffe der allgemeinen Formel (II) sind noch nicht aus der Literatur bekannt; sie sind als neue Stoffe auch Gegenstand der vorliegenden Anmeldung.

Man erhält die neuen Biguanide der allgemeinen Formel (II), wenn man Aminoverbindungen der allgemeinen Formel (IV)

Z-NH₂ (IV)

in welcher
Z die oben angegebene Bedeutung hat,
   - und/oder Säureaddukte von Verbindungen der allgemeinen Formel (IV), wie z.B. die Hydrochloride -
   mit Cyanoguanidin ("Dicyandiamid") der Formel (V) 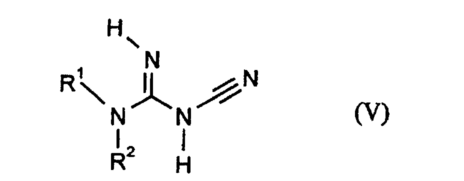
in welcher R¹ und R² die oben angegebene Bedeutung haben,
gegebenenfalls in Gegenwart eines Reaktionshilfsmittels, wie z.B. Hydrogenchlorid, und gegebenenfalls in Gegenwart eines Verdünnungsmittels, wie z.B. n-Decan oder 1,2-Dichlor-benzol, bei Temperaturen zwischen 100°C und 200°C umsetzt (vgl. die Herstellungsbeispiele).

Die Biguanide der allgemeinen Formel (II) können nach ihrer Herstellung auch ohne Zwischenisolierung direkt zur Herstellung der Verbindungen der allgemeinen Formel (I) nach dem erfindungsgemäßen Verfahren eingesetzt werden (vgl. die Herstellungsbeispiele)

Die als Vorprodukte benötigten Aminoverbindungen der allgemeinen Formel (IV) sind bekannt und/oder können nach an sich bekannten Verfahren hergestellt werden (vgl. J. Org. Chem. 18 (1953), 1511-1515; JP-A-03223277 - zitiert in Chem. Abstracts 1992:128652 bzw. 116:128652).

Man erhält die Aminoverbindungen der allgemeinen Formel (IV), wenn man entsprechende cyclische Ketone (einer der Reste A¹, A² oder A³ steht dann für -CO-) mit Formamid bei Temperaturen zwischen 140°C und 190°C umsetzt und die resultierende Formylaminoverbindung anschließend durch Erhitzen mit wässriger Salzsäure hydrolysiert (vgl. J. Org. Chem. 18 (1953), 1511-1515), oder wenn man die entsprechenden cyclischen Ketone zunächst durch Umsetzung mit Hydroxylamin-Hydrochlorid, gegebenenfalls in Gegenwart eines Verdünnungsmittels, wie z.B. Pyridin, bei Temperaturen zwischen 0°C und 50°C in entsprechende Oxime überführt und diese mit einem Reduktionsmittel, wie z.B. Natriumborhydrid, in Gegenwart eines Reaktionshilfsmittels, wie z.B. Titan(IV)-chlorid, und in Gegenwart eines Verdünnungsmittels, wie z.B. 1,2-Dimethoxyethan, bei Temperaturen zwischen - 20°C und +50°C umsetzt (vgl. die Herstellungsbeispiele).

Die entsprechenden cyclischen Ketone sind bekannt und/oder können nach an sich bekannten Verfahren hergestellt werden (vgl. J. Chem. Soc. 1953, 1837-1842; J. Heterocycl. Chem. 2 (1965), 44-48; loc. cit. 17 (1980), 87-92; loc. cit 29 (1992), 1213-1217; J. Pharm. Sci. 52 (1963), 898-901; US-A-3301874).

Die beim erfindungsgemäßen Verfahren zur Herstellung von Verbindungen der allgemeinen Formel (I) weiter als Ausgangsstoffe zu verwendenden Alkoxycarbonyl-Verbindungen sind durch die Formel (III) allgemein definiert. In der allgemeinen Formel (III) hat R³ vorzugsweise bzw. insbesondere diejenige Bedeutung, die bereits oben im Zusammenhang mit der Beschreibung der erfindungsgemäßen Verbindungen der allgemeinen Formel (I) vorzugsweise bzw. als insbesondere bevorzugt für R³ angegeben worden ist; R⁴ steht vorzugsweise für Alkyl mit 1 bis 4 Kohlenstoffatomen, insbesondere für Methyl oder Ethyl.

Die Ausgangsstoffe der allgemeinen Formel (III) sind bekannte Synthesechemikalien.

Das erfindungsgemäße Verfahren zur Herstellung der Verbindungen der Formel (I) wird gegebenenfalls unter Verwendung eines Reaktionshilfsmittels durchgeführt. Als Reaktionshilfsmittel kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kaliumoder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoßverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylaminopyridin, N-Methyl-piperidin, 1,4-Diazabicyclo[2.2.2]-octan (DABCO), 1,5-Diazabicyclo[4.3.0]-non-5-en (DBN), oder 1,8-Diazabicyclo[5.4.0]-undec-7-en (DBU).

Das erfindungsgemäße Verfahren zur Herstellung der Verbindungen der allgemeinen Formel (I) wird gegebenenfalls unter Verwendung eines Verdünnungsmittels durchgeführt. Als Verdünnungsmittel kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Übersehuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvemichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera, Aegilops, Phalaris.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen abergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe fiir Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise

Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzobicyclon, Benzofenap, Benzoylprop(-ethyl), Bialaphos, Bifenox, Bispyribac(sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinidon(-ethyl), Cinmethylin, Cinosulfuron, Clefoxydim, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Fentrazamide, Flamprop(-isopropyl), Flamprop(-isopropyl-L), Flamprop(methyl), Flazasulfuron, Florasulam, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-methyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron(-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffinenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

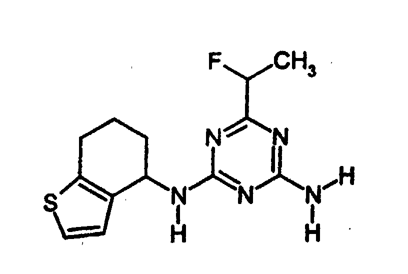

### (Verfahren mit integrierter Herstellung von Ausgangsstoff der Formel (II))

Eine Mischung aus 3,5 g (18,4 mMol) 4,5,6,7-Tetrahydrobenzo[*b*]thiophen-4-ylamin-Hydrochlorid und 1,6 g (18,4 mMol) Cyanoguanidin wird zwei Stunden auf 150°C erhitzt, dann im Aceton-Trockeneisbad abgeschreckt und mit Diethylether verrührt. Der erhaltene kristalline Feststoff wird durch Filtration abgetrennt und in 50 ml Methanol gelöst. Die Lösung wird mit 6,6 g (46,7 mMol) Natriumsulfat versetzt und bei Raumtemperatur (ca. 20°C) werden anschließend nacheinander 1,4 g (13,3 mMol) 2-Fluor-propansäure-methylester und 2,1 g (12,1 mMol) Natriummethylat dazu gegeben. Das Reaktionsgemisch wird 20 Stunden bei Raumtemperatur gerührt und anschließend im Wasserstrahlvakuum eingeengt. Der Rückstand wird zwischen Wasser und Dichlormethan verteilt, die organische Phase abgetrennt, über Natriumsulfat getrocknet und filtriert. Das Filtrat wird im Wasserstrahlvakuum eingeengt und der Rückstand säulenchromatographisch (Kieselgel, Essigsäureethylester/Hexan, Vol.: 20:80) gereinigt.

Man erhält 0,84 g (16 % der Theorie) 2-Amino-4-(1-fluor-ethyl)-6-(4,5,6,7-tetrahydrobenzo[*b*]thiophen-4-yl-amino)-1,3,5-triazin als hellgelbes Öl.
logP = 4,26^{a)}

Analog zu Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) hergestellt werden.

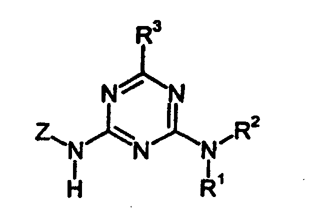

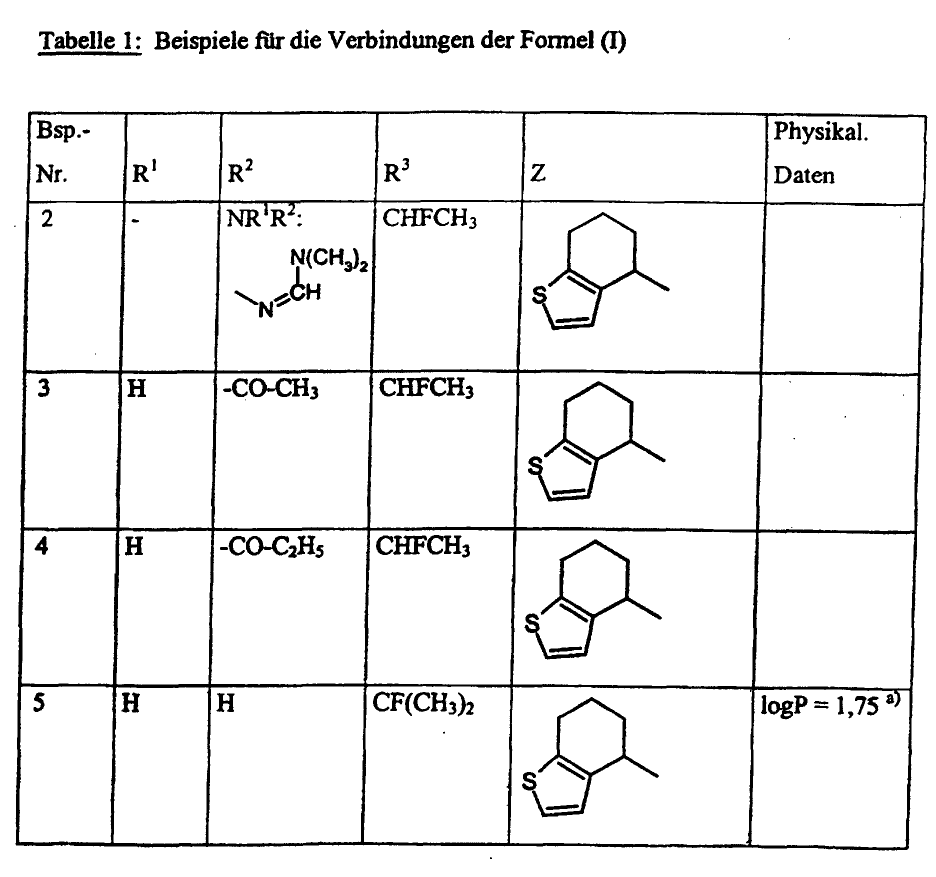

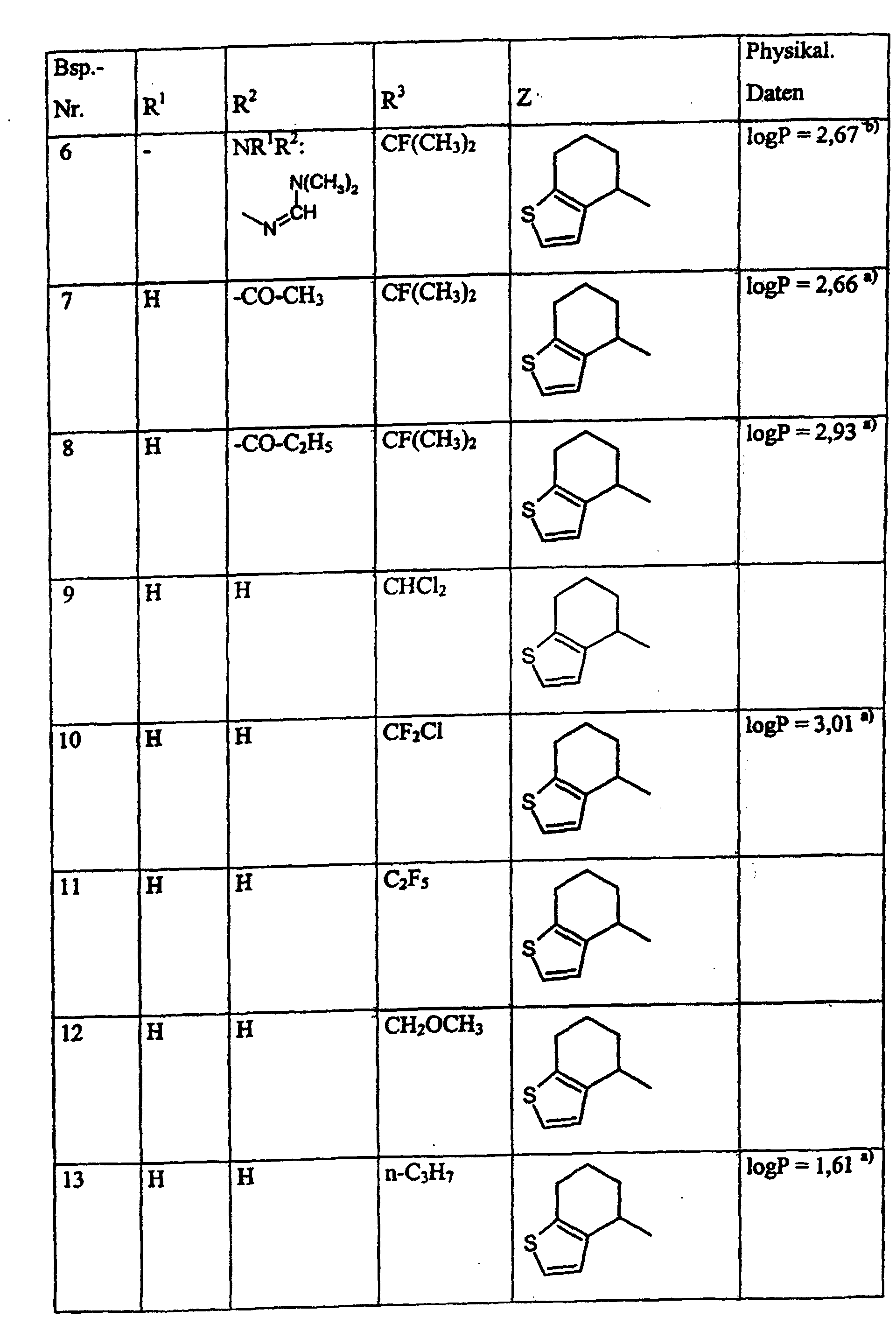

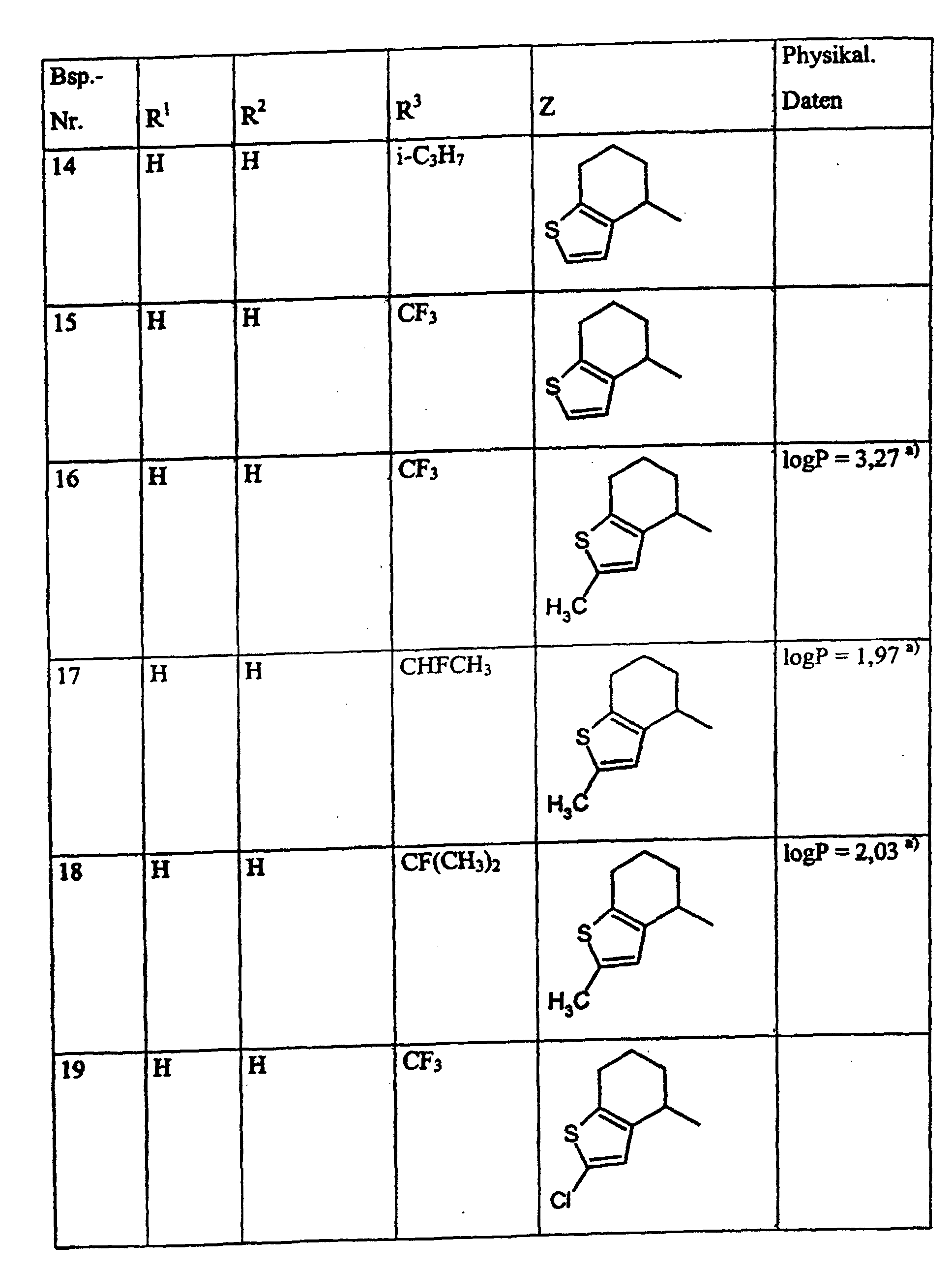

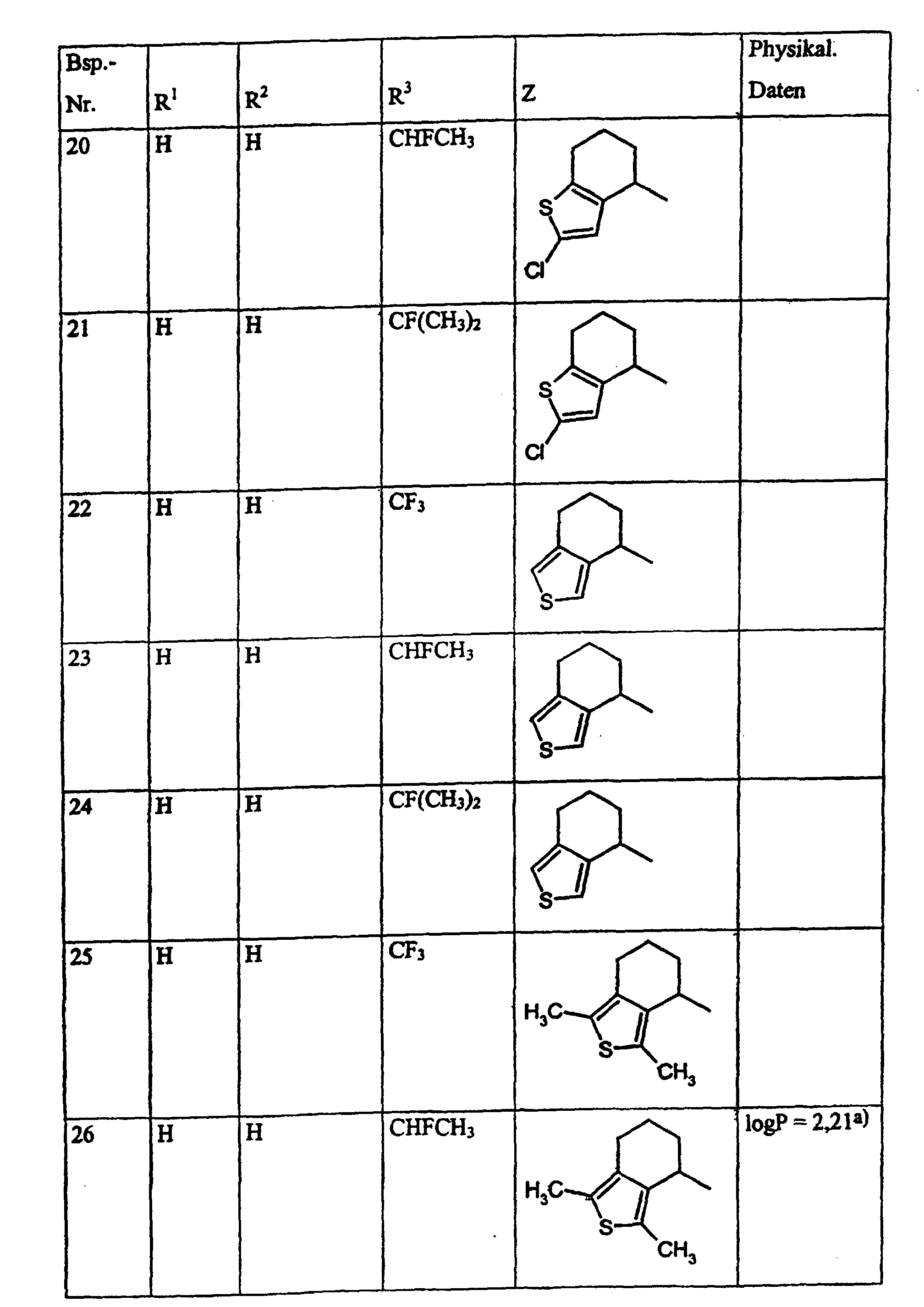

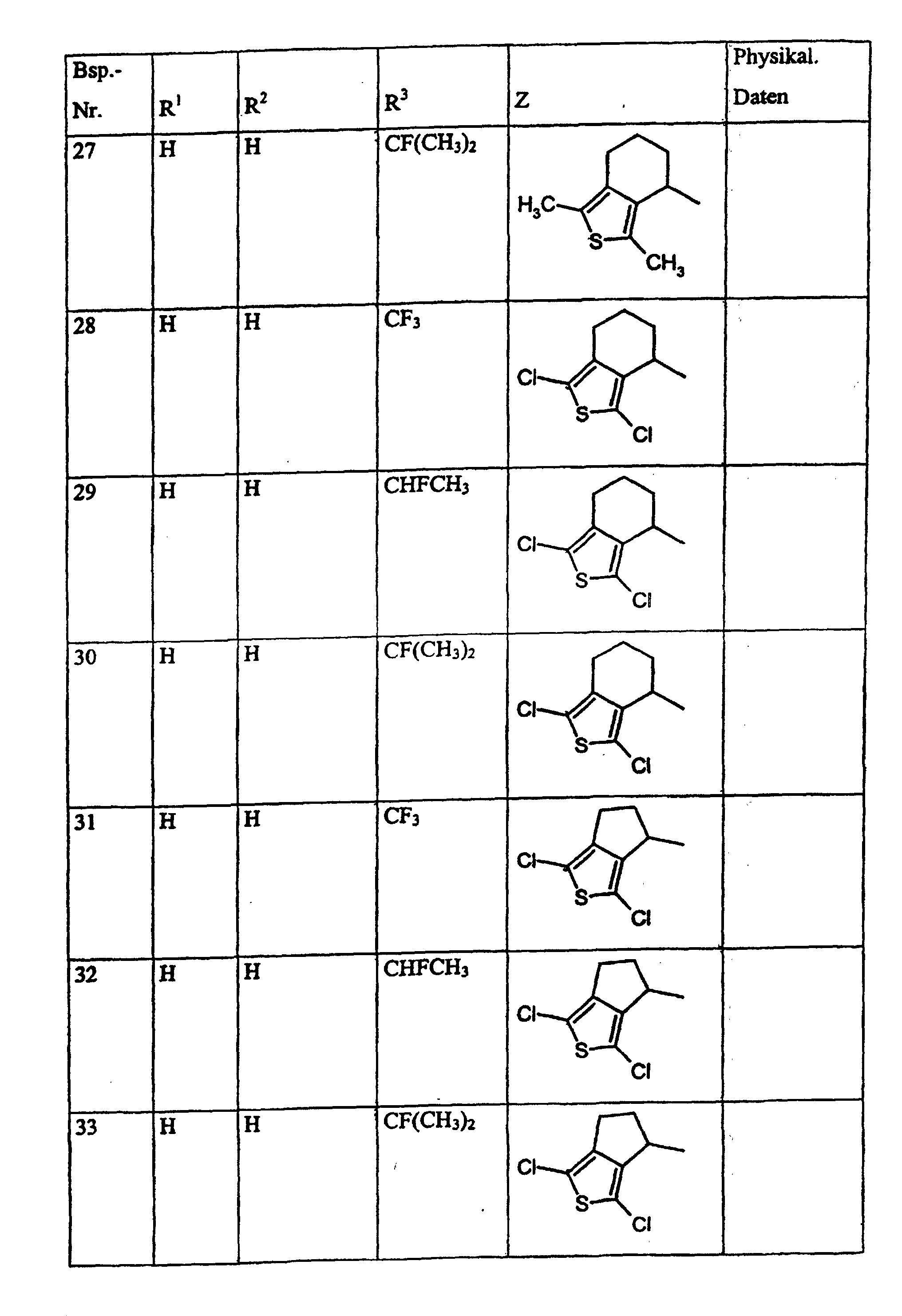

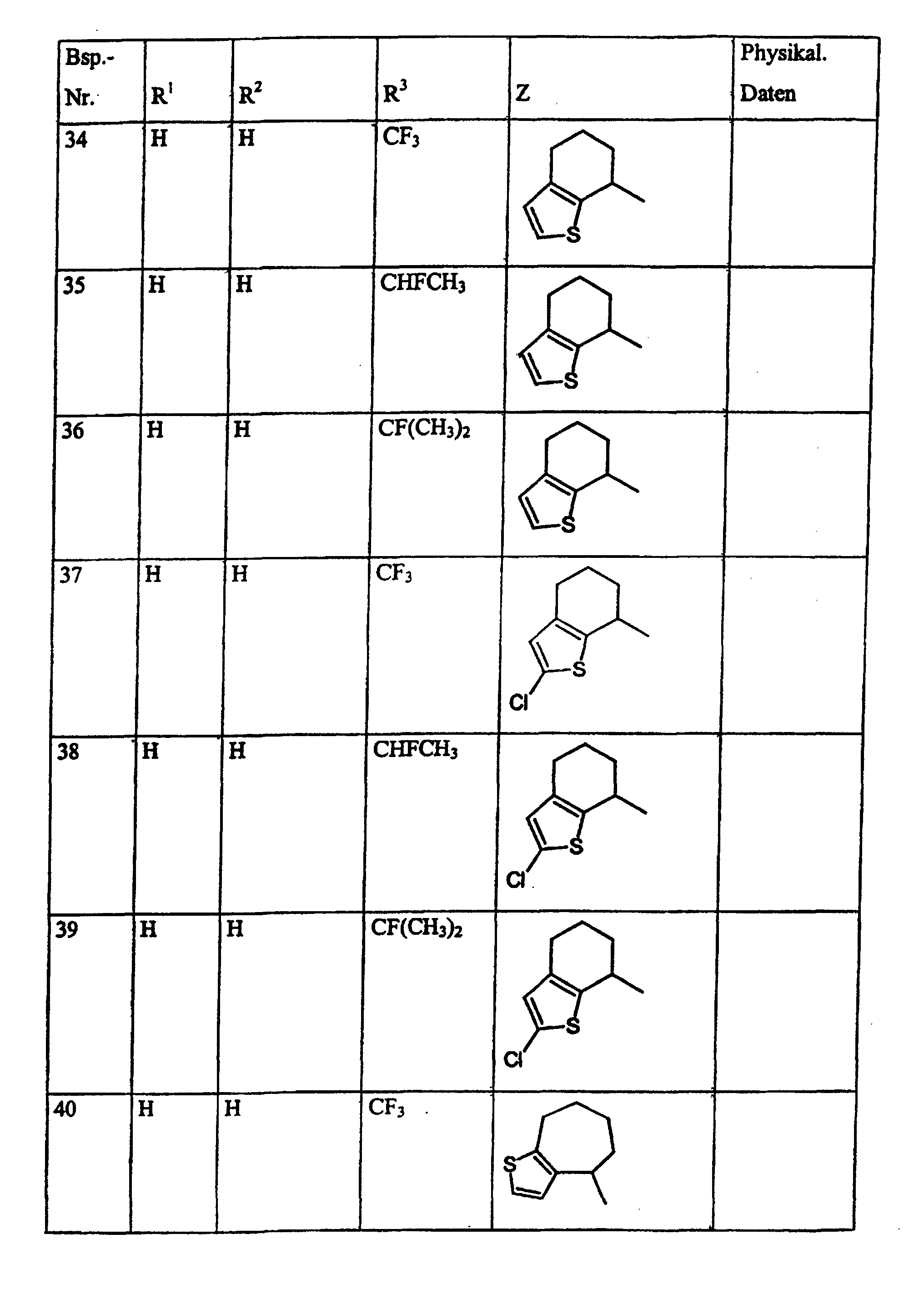

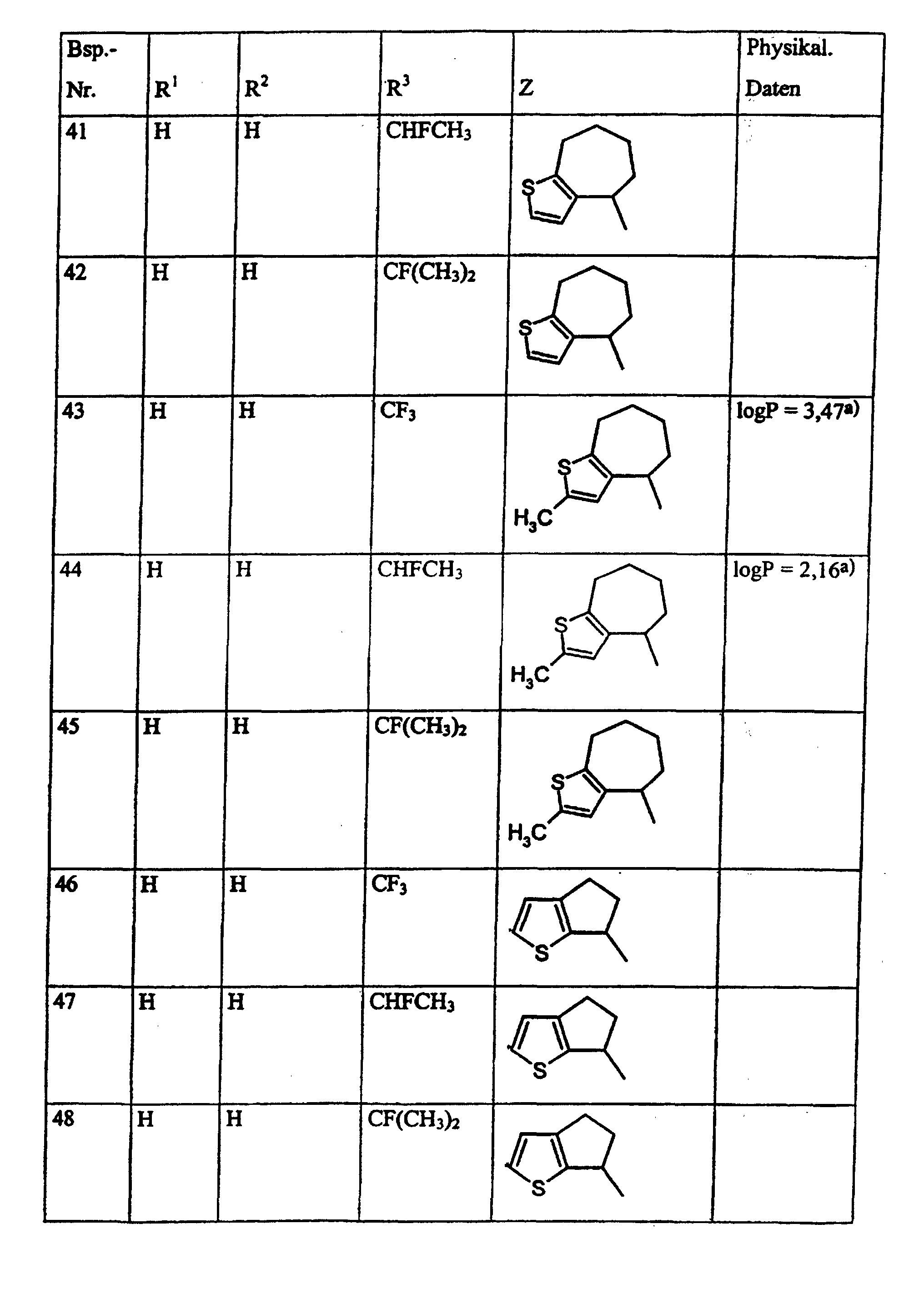

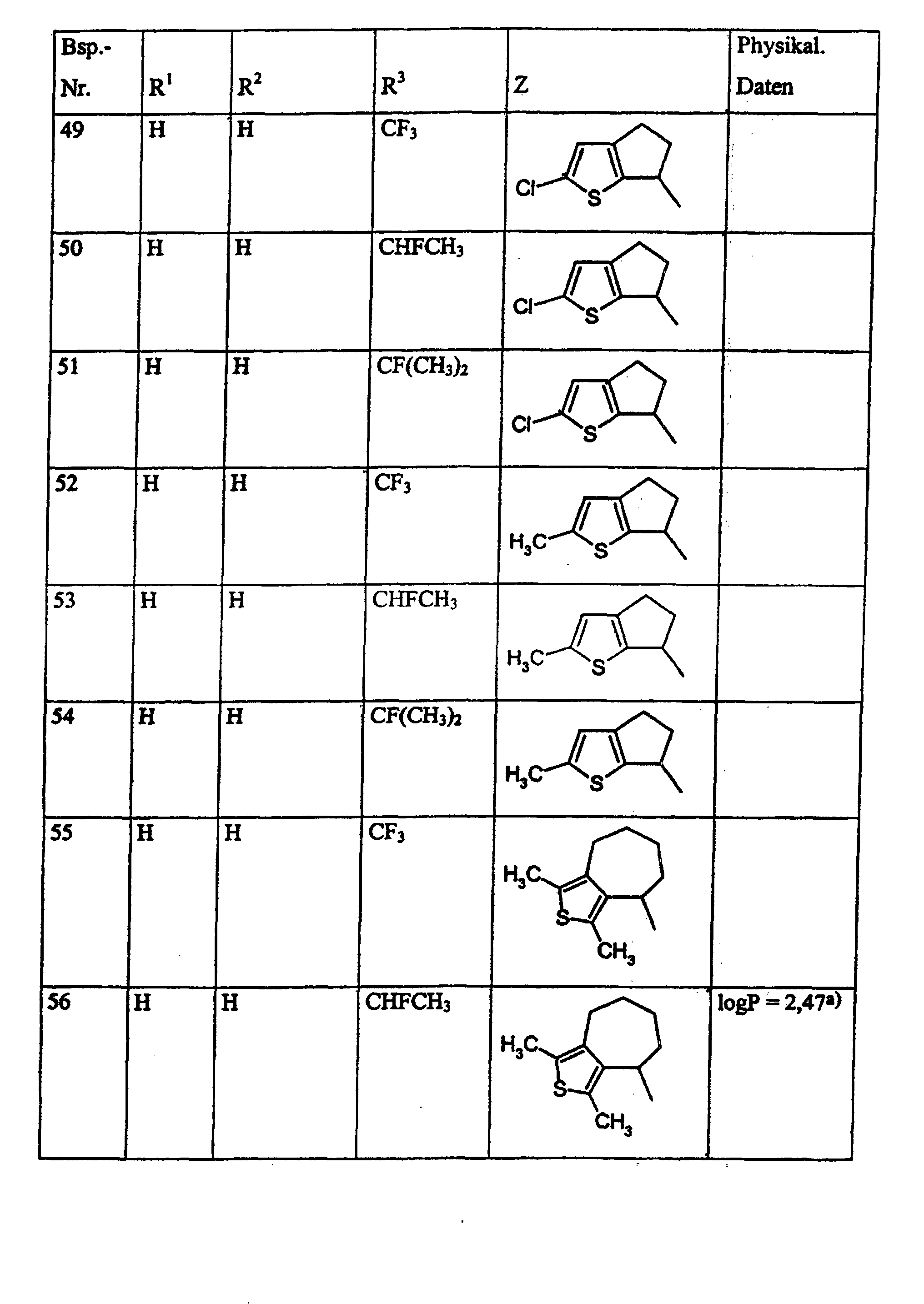

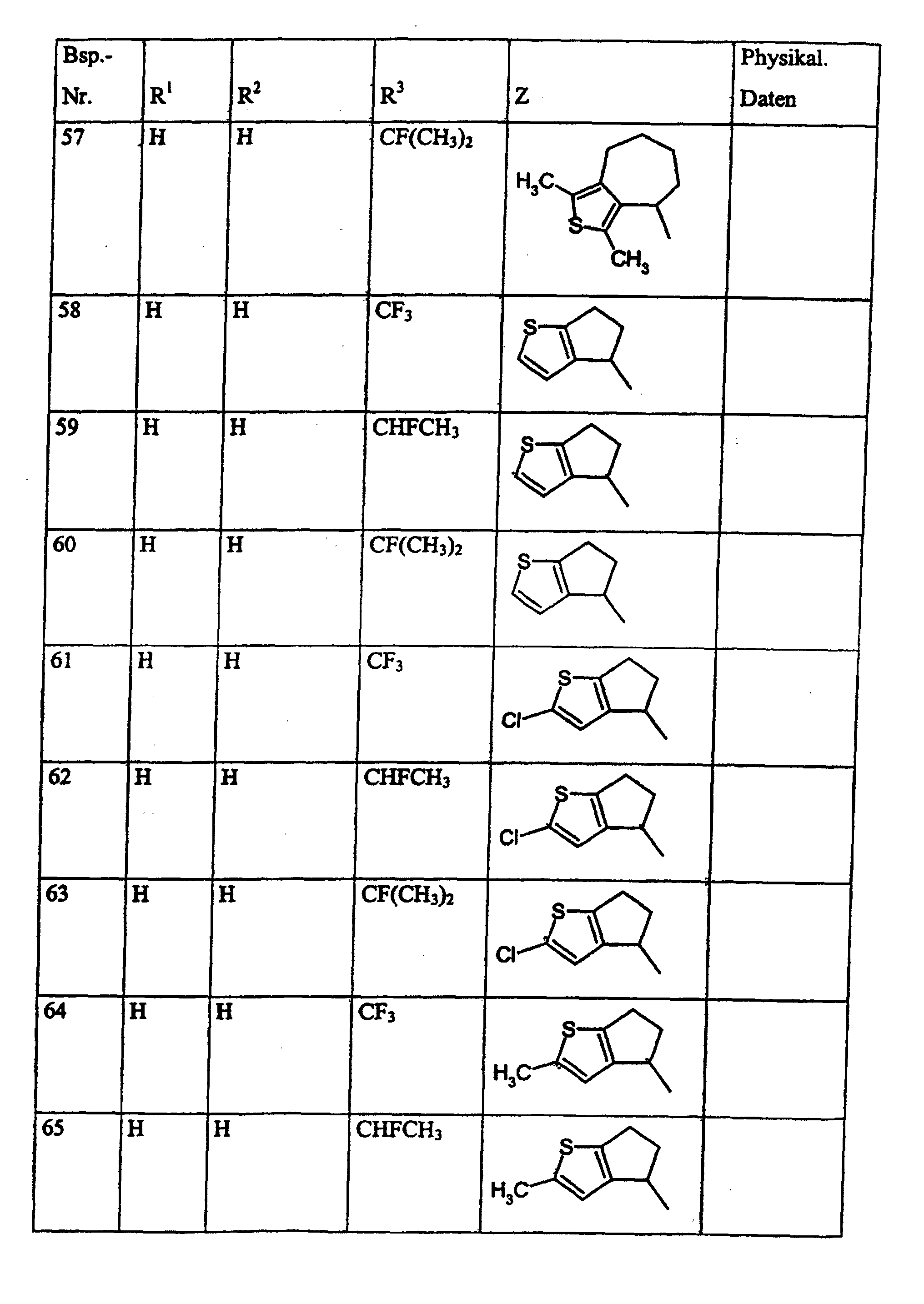

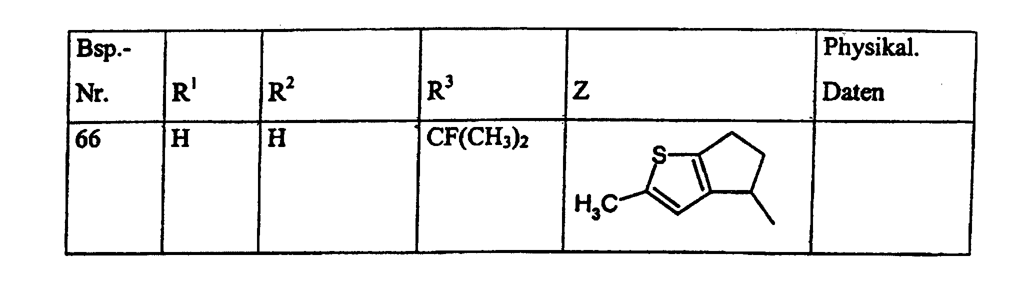

Die Bestimmung der in Beispiel 1 und in Tabelle 1 angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten flir die Bestimmung im sauren Bereich: 0,1% wässrige Phosphorsäure, Acetonitril; linearer Gradient von 10% Acetonitril bis 90% Acetonitril - entsprechende Messergebnisse sind in Tabelle 1 mit ^{a)} markiert.
(b) Eluenten für die Bestimmung im neutralen Bereich: 0,01-molare wässrige Phosphatpuffer-Lösung, Acetonitril; linearer Gradient von 10% Acetonitril bis 90% Acetonitril - entsprechende Messergebnisse sind in Tabelle 1 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Ausgangsstoffe der Formel (II):

### Beispiel II-1

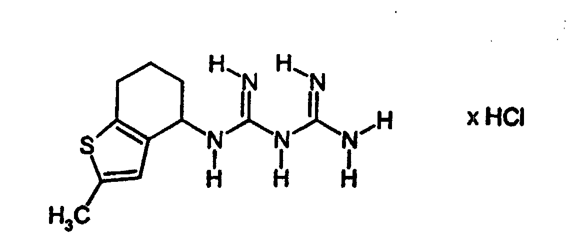

Eine Mischung aus 24,9 g (0,122 Mol) 2-Methyl-4,5,6,7-tetrahydrobenzo[*b*]thiophen-4-yl-amin-Hydrochlorid und 10,3 g (0,122 Mol) Cyanoguanidin wird eine Stunde auf 150°C erhitzt und anschließend im Aceton-Trockeneis-Bad abgeschreckt. Das Reaktionsgemisch wird bei -78°C mit Aceton verrührt und der erhaltene Feststoff wird abfiltriert, bei Zimmertemperatur mit Diethylether verrührt und erneut filtriert.

Man erhält 27,3 g (78 % der Theorie) 2-Methyl-4,5,6,7-tetrahydrobenzo[*b*]thiophen-4-yl-biguanid-Hydrochlorid als dunkelbraunen Feststoff (logP = 1,12 ^{a.)}).

Analog zu Beispiel II-1 können beispielsweise auch die in der nachstehenden Tabelle 2 aufgeführten Verbindungen der allgemeinen Formel (II) hergestellt werden.

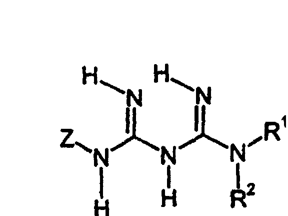

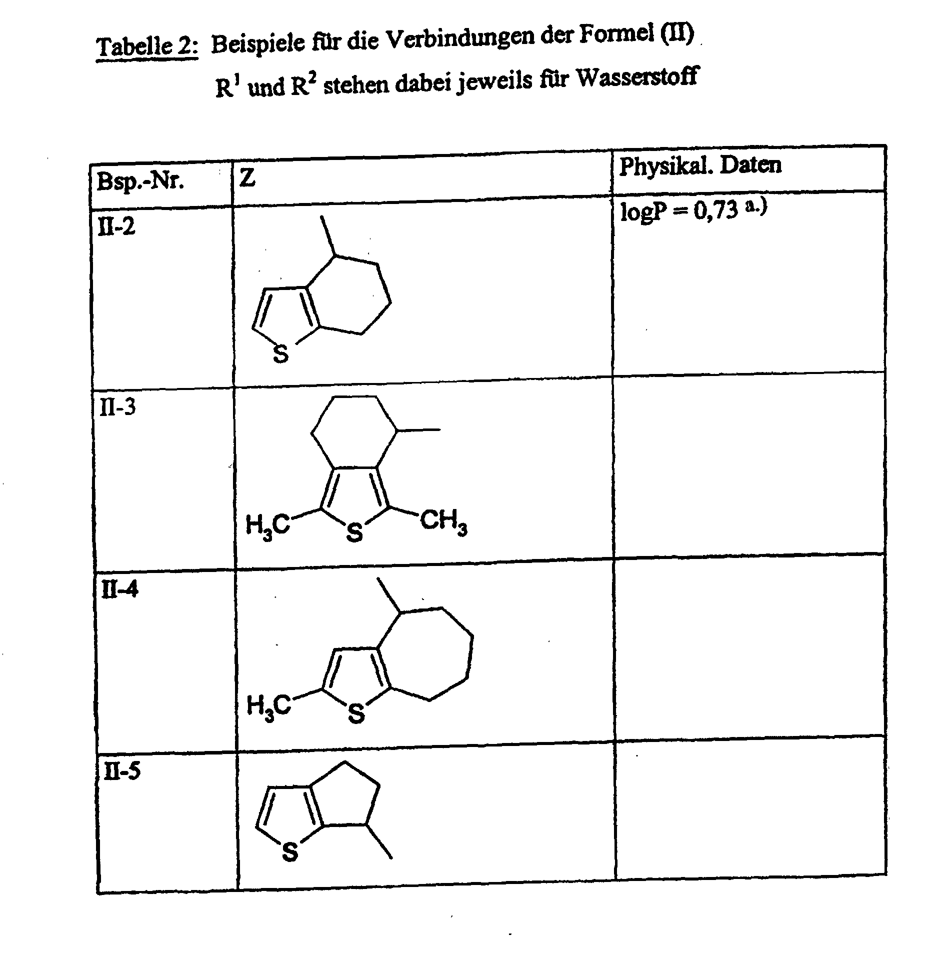

### Ausgangsstoffe der Formel (IV):

### Beispiel IV-1

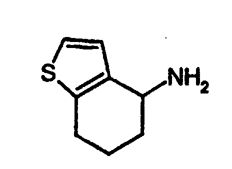

### Stufe 1

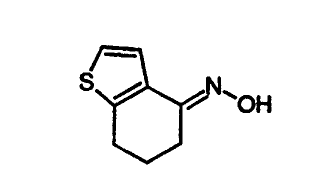

77,3 g (0,51 Mol) 6,7-Dihydro-benzo[*b*]thiophen-4(5*H*)-on werden zusammen mit 69,5 g (1,0 Mol) Hydroxylamin-Hydrochlorid in 600 ml Pyridin zwei Stunden bei Raumtemperatur (ca. 20°C) gerührt. Anschließend wird das Reaktionsgemisch in 1 Liter Wasser eingegossen, mit konz. Salzsäure ein pH-Wert von 1 eingestellt und mit Essigsäureethylester extrahiert. Die organische Extraktionslösung wird über Natriumsulfat getrocknet und filtriert. Das Filtrat wird im Wasserstrahlvakuum eingeengt, der als Feststoff erhaltene Rückstand mit Petrolether verrührt und durch Absaugen isoliert.

Man erhält 74,5 g (88 % der Theorie) 6,7-Dihydro-benzo[*b*]thiophen-4(5*H*)-oxim als 1:1-Mischung der *E*/*Z*-Isomeren.

### Stufe 2

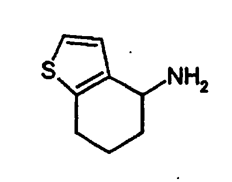

Eine Lösung von 8,4 g (50 mMol) 6,7-Dihydro-benzo[*b*]thiophen-4(5*H*)-oxim in 50 ml 1,2-Dimethoxy-ethan wird zu einer Mischung aus 20,0 g (105 mMol) Titan(IV)-chlorid und 8,0 g (210 mMol) Natriumborhydrid in 200 ml 1,2-Dimethoxy-ethan bei 0°C getropft. Das Reaktionsgemisch wird im Eis-Wasserbad belassen und ca. 20 Stunden gerührt. Zur Aufarbeitung wird auf Wasser gegossen und mit 25%iger Ammoniaklösung ein pH-Wert von 9 eingestellt. Der entstandene Niederschlag wird durch Filtration über Celite abgetrennt und das Filtrat mit Dichlormethan extrahiert. Die organische Extraktionslösung wird über Natriumsulfat getrocknet und filtriert. Das Filtrat wird im Wasserstrahlvakuum eingeengt.

Man erhält 4,5 g (59 % der Theorie) 4,5,6,7-Tetra-hydro-benzo[*b*]thiophen-4-yl-amin als farbloses Öl.

Das Hydrochlorid der gemäß Beispiel IV-1 erhaltenen Verbindung kann beispielsweise wie folgt hergestellt werden:

Eine Mischung aus 4,1 g (27 mMol) 4,5,6,7-Tetrahydro-benzo[*b*]thiophen-4-yl-amin, 4 ml konz. Salzsäure und 50 ml Methanol wird eine Stunde bei Raumtemperatur (ca. 20°C) gerührt und anschließend im Wasserstrahlvakuum eingeengt. Der Rückstand wird mit Diethylether verrührt und das kristallin erhaltene Produkt durch Absaugen isoliert.

Man erhält 3,8 g (75 % der Theorie) 4,5,6,7-Tetrahydro-benzo[*b*]thiophen-4-yl-amin-Hydrochlorid als braunen Feststoff.

### Anwendungsbeispiele:

### Beispiel A

| Pre-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, daß die jeweils gewünschte Wirkstoffmenge pro Flächeneinheit ausgebracht wird. Die Konzentration der Spritzbrühe wird so gewählt, daß in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigt beispielsweise die Verbindung gemäß Herstellungsbeispiel 1 starke Wirkung gegen Unkräuter.

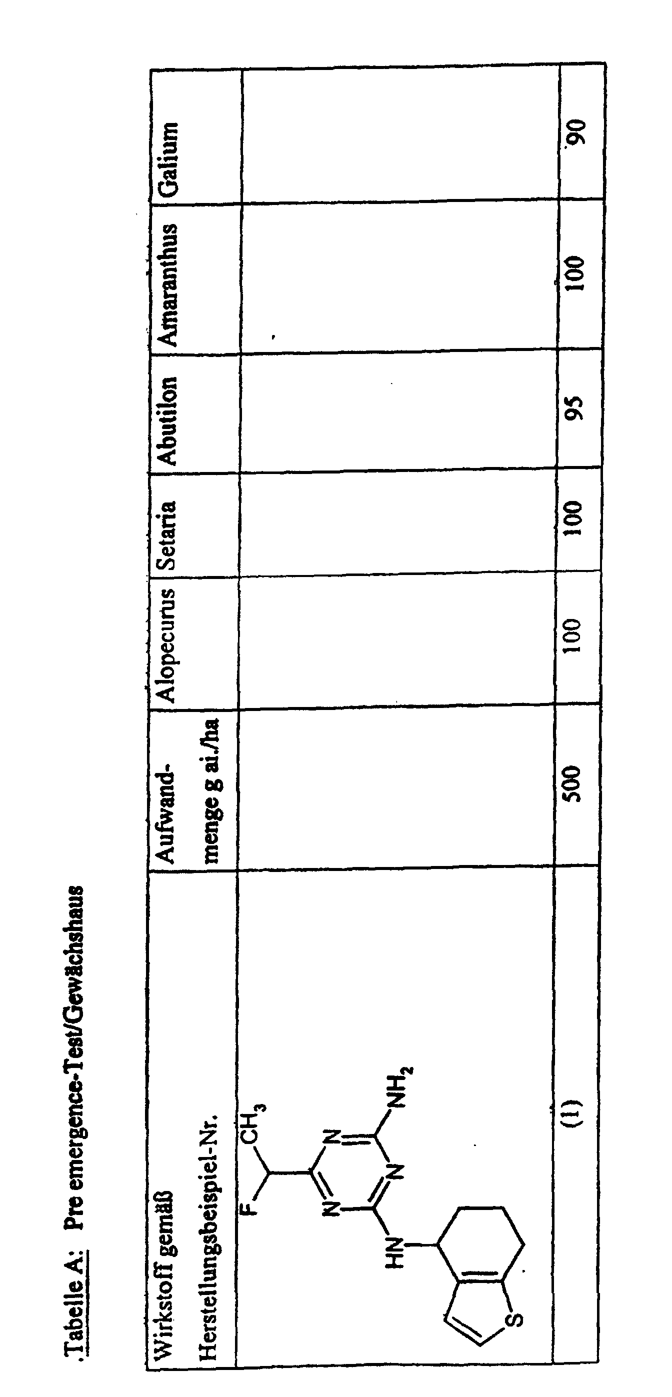

### Beispiel B

| Post-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigt beispielsweise die Verbindung gemäß Herstellungsbeispiel 1 starke Wirkung gegen Unkräuter.

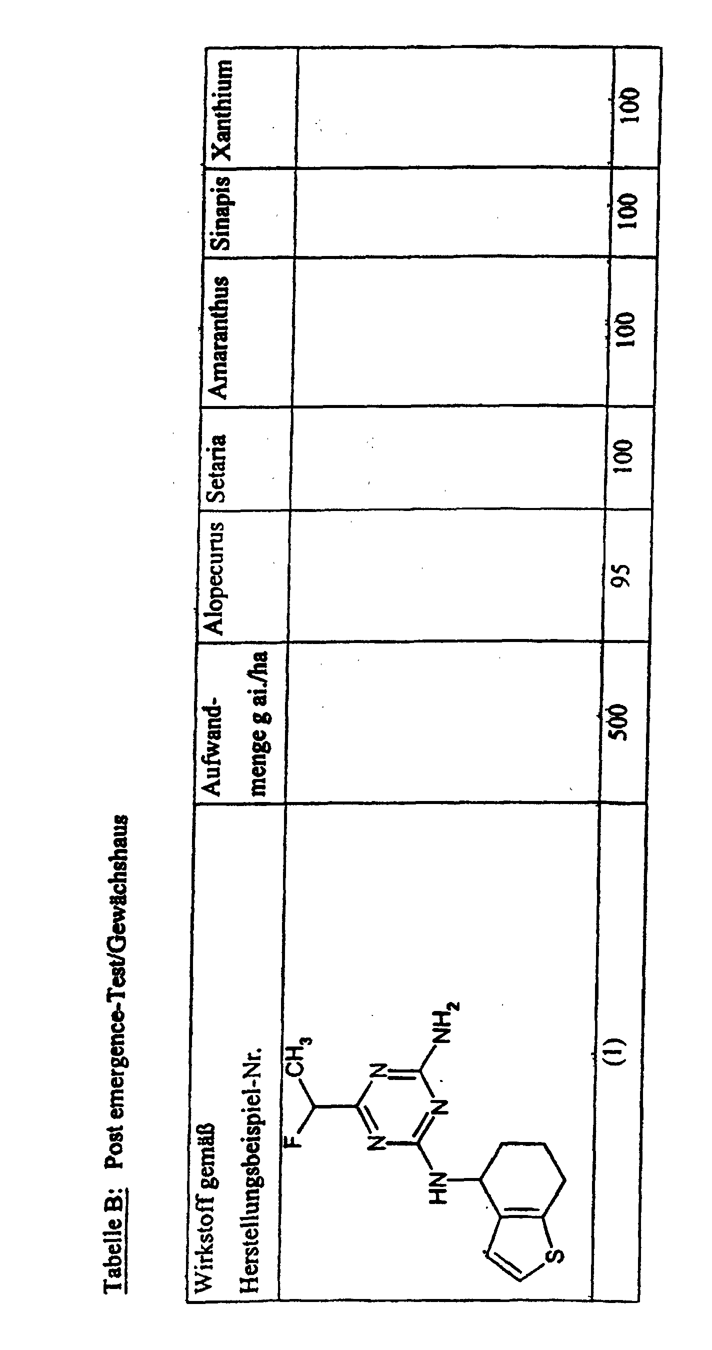

## Patentansprüche

1. Verbindungen der allgemeinen Formel (I) 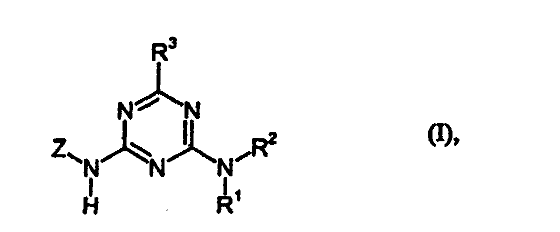 in welcher
R¹ für Wasserstoff oder für gegebenenfalls substituiertes Alkyl steht,
R² für Wasserstoff, für Formyl oder für jeweils gegebenenfalls sub-stituiertes Alkyl, Alkylcarbonyl, Alkoxycarbonyl oder Alkylaminocarbonyl steht,
oder die Gruppierung N(R¹R²) auch für Dialkylaminoalkylidenamino steht,
R³ für Wasserstoff, für Halogen, für gegebenenfalls substituiertes Alkyl, für jeweils gegebenenfalls substituiertes Alkylcarbonyl, Alkoxy-carbonyl, Alkoxy, Alkylthio, Alkylsulfinyl oder Alkylsulfonyl, für jeweils gegebenenfalls substituiertes Alkenyl oder Alkinyl, oder flir gegebenenfalls substituiertes Cycloalkyl steht, und
Z für eine der nachstehenden Thienocycloalk(en)yl-Gruppierungen steht 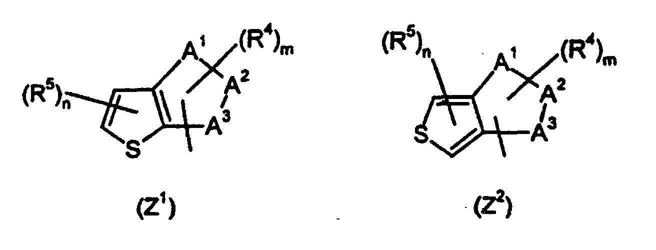
worin
m für die Zahlen 0, 1, 2, 3 oder 4 steht,
n für die Zahlen 0, 1 oder 2 steht,
A¹ für O (Sauerstoff), S (Schwefel), -CO-, -CS- oder Alkandiyl (Alkylen) steht,
A² für O (Sauerstoff), S (Schwefel), -CO-, -CS- oder Alkandiyl (Alkylen) steht,
A³ für O (Sauerstoff), S (Schwefel), -CO-, -CS- oder Alkandiyl (Alkylen) steht,
- mit der Maßgabe, daß von den Gruppierungen A¹, A², A³ wenigstens eine für Alkandiyl steht und nicht zwei benachbarte Gruppen gleichzeitig für S oder O stehen -
R⁴ fiir Amino, Cyano, Carbamoyl, Thiocarbamoyl, Formyl, Halogen, oder für jeweils gegebenenfalls substituiertes Alkyl, Alkylcarbonyl, Alkoxy, Alkoxycarbonyl, Alkylthio, Alkylsulfinyl, Alkylsulfonyl, Alkylamino, Dialkylamino, Alkylcarbonylamino, Alkoxycarbonylamino, Alkylsulfonylamino, Alkenyl, Alkinyl, Alkenylcarbonyl, Alkinylcarbonyl, Aryl, Arylcarbonyl oder Arylalkyl steht, und
R⁵ für Nitro, Amino, Cyano, Carbamoyl, Thiocarbamoyl, Formyl, Halogen, oder für jeweils gegebenenfalls substituiertes Alkyl, Alkylcarbonyl, Alkoxy, Alkoxycarbonyl, Alkylthio, Alkylsulfinyl, Alkylsulfonyl, Alkylamino, Dialkylamino, Alkylcarbonylamino, Alkoxycarbonylamino, Alkylsulfonylamino, Alkenyl, Alkinyl, Alkenylcarbonyl, Alkinylcarbonyl, Aryl, Arylcarbonyl oder Arylalkyl steht.

2. Verbindungen gemäß Anspruch 1, **dadurch gekennzeichnet, daß**
m für die Zahlen 0, 1 oder 2 steht,
A¹ fiir O (Sauerstoff), S (Schwefel), -CO-, -CS- oder Alkandiyl (Alkylen) mit 1 bis 3 Kohlenstoffatomen steht,
A² für O (Sauerstoff), S (Schwefel), -CO-, -CS- oder Alkandiyl (Alkylen) mit 1 bis 3 Kohlenstoffatomen steht,
A³ für O (Sauerstoff), S (Schwefel), -CO-, -CS- oder Alkandiyl (Alkylen) mit 1 bis 3 Kohlenstoffatomen steht,
- mit der Maßgabe, daß von den Gruppierungen A¹, A², A³ wenigstens eine für Alkandiyl mit 1 bis 3 Kohlenstoffatomen steht und zwei benachbarte Gruppen nicht gleichzeitig für S oder O stehen -
R¹ für Wasserstoff oder für gegebenenfalls durch Cyano, Halogen oder C₁-C₄-Alkoxy substituiertes Alkyl mit 1 bis 6 Kohlenstoffatomen steht,
R² für Wasserstoff, für Formyl oder für jeweils gegebenenfalls durch Cyano, Halogen oder C₁-C₄-Alkoxy substituiertes Alkyl, Alkylcarbonyl, Alkoxycarbonyl oder Alkylaminocarbonyl mit jeweils 1 bis 6 Kohlenstoffatomen in den Alkylgruppen steht, oder
die Gruppierung N(R¹R²) für Dialkylaminoalkylidenamino mit jeweils bis zu 4 Kohlenstoffatomen in den Alkylgruppen bzw. Alkylidengruppen steht,
R³ für Wasserstoff, für Halogen, für gegebenenfalls durch Cyano, Halogen, Hydroxy, C₁-C₄-Alkoxy oder C₁-C₄-Alkylthio substituiertes Alkyl mit 1 bis 6 Kohlenstoffatomen, fiir jeweils gegebenenfalls durch Cyano, Halogen oder C₁-C₄-Alkoxy substituiertes Alkylcarbonyl, Alkoxycarbonyl, Alkoxy, Alkylthio, Alkylsulfinyl oder Alkylsulfonyl mit jeweils 1 bis 6 Kohlenstoffatomen in den Alkylgruppen, für jeweils gegebenenfalls durch Halogen substituiertes Alkenyl oder Alkinyl mit jeweils 2 bis 6 Kohlenstoffatomen, oder für gegebenenfalls durch Cyano, Halogen oder C₁-C₄-Alkyl substituiertes Cycloalkyl mit 3 bis 6 Kohlenstoffatomen steht,
R⁴ für Amino, Cyano, Carbamoyl, Thiocarbamoyl, Formyl, Halogen, für jeweils gegebenenfalls durch Cyano, Halogen oder C₁-C₄-Alkoxy substituiertes Alkyl, Alkylcarbonyl, Alkoxy, Alkoxycarbonyl, Alkylthio, Alkylsulfinyl, Alkylsulfonyl, Alkylamino, Dialkylamino, Alkylcarbonylamino, Alkoxycarbonylamino oder Alkylsulfonylamino mit jeweils 1 bis 6 Kohlenstoffatomen in den Alkylgruppen, für jeweils gegebenenfalls durch Cyano oder Halogen substituiertes Alkenyl, Alkinyl, Alkenylcarbonyl oder Alkinylcarbonyl mit jeweils 2 bis 6 Kohlenstoffatomen in den Alkenyl- bzw. Alkinylgruppen, oder für jeweils gegebenenfalls durch Nitro, Cyano, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl, Arylcarbonyl oder Arylalkyl mit jeweils 6 oder 10 Kohlenstoffatomen in der Arylgruppe und gegebenenfalls 1 bis 4 Kohlenstoffatomen im Alkylteil steht, und
R⁵ für Nitro, Amino, Cyano, Carbamoyl, Thiocarbamoyl, Formyl, Halogen, für jeweils gegebenenfalls durch Cyano, Halogen oder C₁-C₄-Alkoxy substituiertes Alkyl, Alkylcarbonyl, Alkoxy, Alkoxycarbonyl, Alkylthio, Alkylsulfinyl, Alkylsulfonyl, Alkylamino, Dialkylamino, Alkylcarbonylamino, Alkoxycarbonylamino oder Alkylsulfonylamino mit jeweils 1 bis 6 Kohlenstoffatomen in den Alkylgruppen, für jeweils gegebenenfalls durch Cyano oder Halogen substituiertes Alkenyl, Alkinyl, Alkenylcarbonyl oder Alkinylcarbonyl mit jeweils 2 bis 6 Kohlenstoffatomen in den Alkenyl- bzw. Alkinylgruppen, oder für jeweils gegebenenfalls durch Nitro, Cyano, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl, Arylcarbonyl oder Arylalkyl mit jeweils 6 oder 10 Kohlenstoffatomen in der Arylgruppe und gegebenenfalls 1 bis 4 Kohlenstoffatomen im Alkylteil steht.

3. Verbindungen gemäß Anspruch 1 oder 2, **dadurch gekennzeichnet, daß**
A¹ für O (Sauerstoff), S (Schwefel), -CO-, -CS-, Methylen, Dimethylen oder Trimethylen steht,
A² für O (Sauerstoff), S (Schwefel), -CO-, -CS-, Methylen, Dimethylen oder Trimethylen steht,
A³ für O (Sauerstoff), S (Schwefel), -CO-, -CS-, Methylen, Dimethylen oder Trimethylen steht,
- mit der Maßgabe, daß von den Gruppierungen A¹, A², A³ wenigstens eine für Methylen, Dimethylen oder Trimethylen steht und zwei benachbarte Gruppen nicht gleichzeitig für S oder O stehen -
R¹ für Wasserstoff oder für jeweils gegebenenfalls durch Cyano, Fluor, Chlor, Methoxy oder Ethoxy substituiertes Methyl, Ethyl, n- oder i-Propyl steht,
R² für Wasserstoff, für Formyl oder flir jeweils gegebenenfalls durch Cyano, Fluor, Chlor, Methoxy oder Ethoxy substituiertes Methyl, Ethyl, n- oder i-Propyl, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl steht, oder
die Gruppierung N(R¹R²) für Dimethylaminomethylenamino oder Diethylaminomethylenamino steht,
R³ für Wasserstoff, für Fluor, Chlor, Brom, für jeweils gegebenenfalls durch Cyano, Fluor, Chlor, Brom, Hydroxy, Methoxy, Ethoxy, n- oder i-Propoxy, Methylthio, Ethylthio, n- oder i-Propylthio substituiertes Methyl, Ethyl, n- oder i-Propyl, n-, i- oder s-Butyl, für jeweils gegebenenfalls durch Cyano, Fluor, Chlor, Methoxy oder Ethoxy substituiertes Acetyl, Propionyl, n- oder i-Butyroyl, Methoxycarbonyl, Ethoxycarbonyl, n- oder i-Propoxycarbonyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, für jeweils gegebenenfalls durch Fluor, Chlor oder Brom substituiertes Ethenyl, Propenyl, Butenyl, Ethinyl, Propinyl oder Butinyl, oder für jeweils gegebenenfalls durch Cyano, Fluor, Chlor, Methyl oder Ethyl substituiertes Cyclopropyl, Cyclobutyl, Cyclopentyl oder Cyclohexyl steht,
R⁴ für Amino, Cyano, Carbamoyl, Thiocarbamoyl, Formyl, Fluor, Chlor, Brom, für jeweils gegebenenfalls durch Cyano, Fluor, Chlor, Methoxy oder Ethoxy substituiertes Methyl, Ethyl, n- oder i-Propyl, Acetyl, Propionyl, n- oder i-Butyroyl, Methoxy, Ethoxy, n- oder i-Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, n- oder i-Propylsulfinyl, Methylsulfonyl, Ethylsulfonyl, n-oder i-Propylsulfonyl, Methylamino, Ethylamino, n- oder i-Propylamino, Dimethylamino, Diethylamino, Acetylamino, Propionylamino, n- oder i-Butyroylamino, Methoxycarbonylamino, Ethoxycarbonylamino, n- oder i-Propoxycarbonylamino, Methylsulfonylamino, Ethylsulfonylamino, n- oder i-Propylsulfonylamino, für jeweils gegebenenfalls durch Cyano, Fluor, Chlor oder Brom substituiertes Ethenyl, Propenyl, Butenyl, Ethinyl, Propinyl, Butinyl, Ethenylcarbonyl, Propenylcarbonyl, Butenylcarbonyl, Ethinylcarbonyl, Propinylcarbonyl oder Butinylcarbonyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluomiethoxy, Trifluormethoxy, Methoxycarbonyl, Ethoxycarbonyl, n- oder i-Propoxycarbonyl substituiertes Phenyl, Benzoyl oder Benzyl steht, und
R⁵ für Nitro, Amino, Cyano, Carbamoyl, Thiocarbamoyl, Formyl, Fluor, Chlor, Brom, für jeweils gegebenenfalls durch Cyano, Fluor, Chlor, Methoxy oder Ethoxy substituieztes Methyl, Ethyl, n- oder i-Propyl, Acetyl, Propionyl, n- oder i-Butyroyl, Methoxy, Ethoxy, n- oder i-Propoxy, Methoxycarbonyl, Ethoxycarbonyl, n- oder i-Propoxycarbonyl, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, Dimethylamino, Diethylamino, Acetylamino, Propionylamino, n- oder i-Butyroylamino, Methoxycarbonylamino, Ethoxycarbonylamino, n- oder i-Propoxycarbonylamino, Methylsulfonylamino, Ethylsulfonylamino, n- oder i-Propylsulfonylamino, für jeweils gegebenenfalls durch Cyano, Fluor, Chlor oder Brom substituiertes Ethenyl, Propenyl, Butenyl, Ethinyl, Propinyl, Butinyl, Ethenylcarbonyl, Propenylcarbonyl, Butenylcarbonyl, Ethinylcarbonyl, Propinylcarbonyl oder Butinylcarbonyl, oder flir jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methoxycarbonyl, Ethoxycarbonyl, n- oder i-Propoxycarbonyl substituiertes Phenyl, Benzoyl oder Benzyl steht.

4. Verbindungen gemäß einem der Ansprüche 1 bis 3, **dadurch gekennzeichnet, daß**
A¹ für Methylen oder Dimethylen steht,
A² für Methylen oder Dimethylen steht,
A³ für Methylen oder Dimethylen steht,
R¹ für Wasserstoff steht,
R² für Wasserstoff, für Formyl oder für jeweils gegebenenfalls durch Fluor, Chlor, Methoxy oder Ethoxy substituiertes Acetyl, Propionyl, n- oder i-Butyroyl, Methoxycarbonyl, Ethoxycarbonyl, n- oder i-Propoxycarbonyl steht, oder
die Gruppierung N(R¹R²) für Dimethylaminomethylenamino steht,
R³ für jeweils gegebenenfalls durch Fluor oder Chlor substituiertes Methyl, Ethyl, n- oder i-Propyl steht,
R⁴ für Cyano, Fluor, Chlor, Brom, oder für jeweils gegebenenfalls durch Fluor oder Chlor substituiertes Methyl, Ethyl, Methoxy oder Ethoxy steht, und
R⁵ für Nitro, Cyano, Fluor, Chlor, Brom, oder für jeweils gegebenenfalls durch Fluor oder Chlor substituiertes Methyl, Ethyl, Methoxy oder Ethoxy steht.

5. Verbindungen gemäß einem der Ansprüche 1 bis 4, **dadurch gekennzeichnet, daß**
Z für 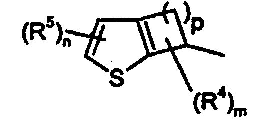 steht,
wobei
p für 2, 3 oder 4 steht und n, m, R⁴ und R⁵ die in einem der Ansprüche 1 bis 4 angegebene Bedeutung haben.

6. Verfahren zum Herstellen von substituierten Triazinen gemäß einem der Ansprüche 1 bis 5, **dadurch gekennzeichnet, daß** Biguanide der allgemeinen Formel (II) 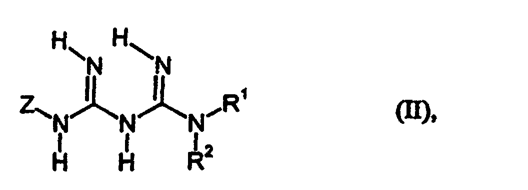 in welcher
R¹, R² und Z die in einem der Ansprüche 1 bis 5 angegebene Bedeutung haben,
und/oder Säureaddukte von Verbindungen der allgemeinen Formel (II)
mit Alkoxycarbonylverbindungen der allgemeinen Formel (III)
R³-CO-OR⁴ (III)
in welcher
R³ die in einem der Ansprüche 1 bis 4 angebene Bedeutung hat und
R⁴ für Alkyl steht,
gegebenenfalls in Gegenwart eines Reaktionshilfsmittels und gegebenenfalls in Gegenwart eines Verdünnungsmittels umgesetzt werden und gegebenenfalls an den so erhaltenen Verbindungen der allgemeinen Formel (I) im Rahmen der Substituentendefinition weitere Umwandlungen nach üblichen Methoden durchgeführt werden.

7. Verbindungen der Formel (II) 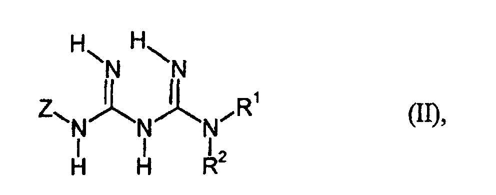 **dadurch gekennzeichnet, dass**
R¹, R² und Z die in einem der Ansprüche 1 bis 5 angegebene Bedeutung haben,
sowie die Säureaddukte der Verbindungen der allgemeinen Formel (II).

8. Verfahren zum Herstellen von Verbindungen gemäß Anspruch 7, **dadurch gekennzeichnet, daß** Aminoverbindungen der allgemeinen Formel (IV)
Z-NH₂ (IV)
in welcher
Z die in einem der Ansprüche 1 bis 5 angegebene Bedeutung hat,
und/oder Säureaddukte von Verbindungen der allgemeinen Formel (IV)
mit Cyanoguanidin der Formel (V) 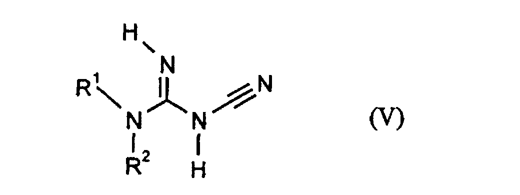 in welcher R¹ und R² die in Anspruch 1 angegebene Bedeutung haben, gegebenenfalls in Gegenwart eines Reaktionshilfsmittels und gegebenenfalls in Gegenwart eines Verdünnungsmittels bei Temperaturen zwischen 100°C und 200°C umgesetzt werden.

9. Verfahren zum Bekämpfen von unerwünschtem Pflanzenwuchs, **dadurch gekennzeichnet, daß** man mindestens eine Verbindung gemäß einem der Ansprüche 1 bis 5 auf unerwünschte Pflanzen und/oder ihren Lebensraum einwirken läßt.

10. Verwendung von mindestens einer Verbindung gemäß einem der Ansprüche 1 bis 5 zum Bekämpfen von unerwünschten Pflanzen.

11. Herbizides Mittel, **gekennzeichnet durch** einen Gehalt an einer Verbindung gemäß einem der Ansprüche 1 bis 5 und üblichen Streckmitteln und/oder oberflächenaktiven Mitteln.

## Claims

1. Compounds of the general formula (I) 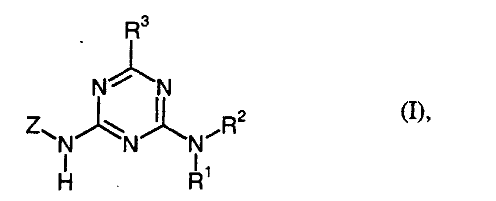 in which
R¹ represents hydrogen or represents optionally substituted alkyl,
R² represents hydrogen, represents formyl or represents in each case optionally substituted alkyl, alkylcarbonyl, alkoxycarbonyl or alkylaminocarbonyl,
or the grouping N(R¹R²) also represents dialkylaminoalkylideneamino,
R³ represents hydrogen, represents halogen, represents optionally substituted alkyl, represents in each case optionally substituted alkylcarbonyl, alkoxycarbonyl, alkoxy, alkylthio, alkylsulphinyl or alkylsulphonyl, represents in each case optionally substituted alkenyl or alkinyl, or represents optionally substituted cycloalkyl, and
Z represents one of the thienocycloalk(en)yl groupings below 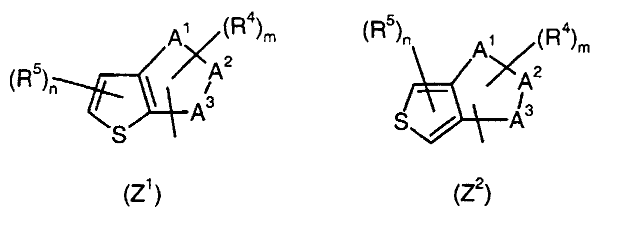 in which
m represents the numbers 0, 1, 2, 3 or 4,
n represents the numbers 0, 1 or 2,
A¹ represents O (oxygen), S (sulphur), -CO-, -CS- or alkanediyl (alkylene),
A² represents O (oxygen), S (sulphur), -CO-, -CS- or alkanediyl (alkylene),
A³ represents O (oxygen), S (sulphur), -CO-, -CS- or alkanediyl (alkylene),
- with the proviso that at least one of the groupings A¹, A², A³ represents alkanediyl and that two adjacent groups do not simultaneously represent S or O -
R⁴ represents amino, cyano, carbamoyl, thiocarbamoyl, formyl, halogen, or represents in each case optionally substituted alkyl, alkylcarbonyl, alkoxy, alkoxycarbonyl, alkylthio, alkylsulphinyl, alkylsulphonyl, alkylamino, dialkylamino, alkylcarbonylamino, alkoxycarbonylamino, alkylsulphonylamino, alkenyl, alkinyl, alkenylcarbonyl, alkinylcarbonyl, aryl, arylcarbonyl or arylalkyl, and
R⁵ represents nitro, amino, cyano, carbamoyl, thiocarbamoyl, formyl, halogen, or represents in each case optionally substituted alkyl, alkylcarbonyl, alkoxy, alkoxycarbonyl, alkylthio, alkylsulphinyl, alkylsulphonyl, alkylamino, dialkylamino, alkylcarbonylamino, alkoxycarbonylamino, alkylsulphonylamino, alkenyl, alkinyl, alkenylcarbonyl, alkinylcarbonyl, aryl, arylcarbonyl orarylalkyl.

2. Compounds according to Claim 1, **characterized in that**
m represents the numbers 0, 1 or 2,
A¹ represents O (oxygen), S (sulphur), -CO-, -CS- or alkanediyl (alkylene) having 1 to 3 carbon atoms,
A² represents O (oxygen), S (sulphur), -CO-, -CS- or alkanediyl (alkylene) having 1 to 3 carbon atoms,
A³ represents O (oxygen), S (sulphur), -CO-, -CS- or alkanediyl (alkylene) having 1 to 3 carbon atoms,
- with the proviso that at least one of the groupings A¹, A², A³ represents alkanediyl having 1 to 3 carbon atoms and that two adjacent groups do not simultaneously represent S or O -
R¹ represents hydrogen or represents optionally cyano-, halogen- or C₁-C₄-alkoxy-substituted alkyl having 1 to 6 carbon atoms,
R² represents hydrogen, represents formyl or represents in each case optionally cyano-, halogen- or C₁-C₄-alkoxy-substituted alkyl, alkylcarbonyl, alkoxycarbonyl or alkylaminocarbonyl having in each case 1 to 6 carbon atoms in the alkyl groups, or
the grouping N(R¹R²) represents dialkylaminoalkylideneamino having in each case up to 4 carbon atoms in the alkyl groups or alkylidene groups,
R³ represents hydrogen, represents halogen, represents optionally cyano-, halogen-, hydroxyl-, C₁-C₄-alkoxy- or C₁-C₄-alkylthio-substituted alkyl having 1 to 6 carbon atoms, represents in each case optionally cyano-, halogen- or C₁-C₄-alkoxy-substituted alkylcarbonyl, alkoxycarbonyl, alkoxy, alkylthio, alkylsulphinyl or alkylsulphonyl having in each case 1 to 6 carbon atoms in the alkyl groups, represents in each case optionally halogen-substituted alkenyl or alkinyl having in each case 2 to 6 carbon atoms, or represents optionally cyano-, halogen- or C₁-C₄-alkyl-substituted cycloalkyl having 3 to 6 carbon atoms,
R⁴ represents amino, cyano, carbamoyl, thiocarbamoyl, formyl, halogen, represents in each case optionally cyano-, halogen- or C₁-C₄-alkoxy-substituted alkyl, alkylcarbonyl, alkoxy, alkoxycarbonyl, alkylthio, alkylsulphinyl, alkylsulphonyl, alkylamino, dialkylamino, alkylcarbonylamino, alkoxycarbonylamino or alkylsulphonylamino having in each case 1 to 6 carbon atoms in the alkyl groups, represents in each case optionally cyano- or halogen-substituted alkenyl, alkinyl, alkenylcarbonyl or alkinylcarbonyl having in each case 2 to 6 carbon atoms in the alkenyl or alkinyl groups, or represents in each case optionally nitro-, cyano-, halogen-, C₁-C₄-alkyl-, C₁-C₄-halogenoalkyl-, C₁-C₄-alkoxy-, C₁-C₄-halogenoalkoxy- or C₁-C₄-alkoxy-carbonyl-substituted aryl, arylcarbonyl or arylalkyl having in each case 6 or 10 carbon atoms in the aryl group and optionally 1 to 4 carbon atoms in the alkyl moiety, and
R⁵ represents nitro, amino, cyano, carbamoyl, thiocarbamoyl, formyl, halogen, represents in each case optionally cyano-, halogen- or C₁-C₄-alkoxy-substituted alkyl, alkylcarbonyl, alkoxy, alkoxycarbonyl, alkylthio, alkylsulphinyl, alkylsulphonyl, alkylamino, dialkylamino, alkylcarbonylamino, alkoxycarbonylamino or alkylsulphonylamino having in each case 1 to 6 carbon atoms in the alkyl groups, represents in each case optionally cyano- or halogen-substituted alkenyl, alkinyl, alkenylcarbonyl or alkinylcarbonyl having in each case 2 to 6 carbon atoms in the alkenyl or alkinyl groups, or represents in each case optionally nitro-, cyano-, halogen-, C₁-C₄-alkyl-, C₁-C₄-halogenoalkyl-, C₁-C₄-alkoxy-, C₁-C₄-halogenoalkoxy- or C₁-C₄-alkoxy-carbonyl substituted aryl, arylcarbonyl or arylalkyl having in each case 6 or 10 carbon atoms in the aryl group and optionally 1 to 4 carbon atoms in the alkyl moiety.

3. Compounds according to Claim 1 or 2, **characterized in that**
A¹ represents O (oxygen), S (sulphur), -CO-, -CS-, methylene, dimethylene or trimethylene,
A² represents O (oxygen), S (sulphur), -CO-, -CS-, methylene, dimethylene or trimethylene,
A³ represents O (oxygen), S (sulphur), -CO-, -CS-, methylene, dimethylene or trimethylene,
- with the proviso that at least one of the groupings A¹, A², A³ represents methylene, dimethylene or trimethylene and that two adjacent groups do not simultaneously represent S or O -
R¹ represents hydrogen or represents in each case optionally cyano-, fluorine-, chlorine-, methoxy- or ethoxy-substituted methyl, ethyl, n- or i-propyl,
R² represents hydrogen, represents formyl or represents in each case optionally cyano-, fluorine-, chlorine-, methoxy- or ethoxy-substituted methyl, ethyl, n- or i-propyl, acetyl, propionyl, n- or i-butyroyl, methoxycarbonyl, ethoxycarbonyl, n- or i-propoxycarbonyl, methylaminocarbonyl, ethylaminocarbonyl, n- or i-propylaminocarbonyl, or
the grouping N(R¹R²) represents dimethylaminomethyleneamino or di-ethylaminomethyleneamino,
R³ represents hydrogen, represents fluorine, chlorine, bromine, represents in each case optionally cyano-, fluorine-, chlorine-, bromine-, hydroxyl-, methoxy-, ethoxy-, n- or i-propoxy-, methylthio-, ethylthio-, n- or i-propylthio-substituted methyl, ethyl, n- or i-propyl, n-, i- or s-butyl, represents in each case optionally cyano-, fluorine-, chlorine-, methoxy- or ethoxy- substituted acetyl, propionyl, n- or i-butyroyl, methoxycarbonyl, ethoxycarbonyl, n- or i-propoxycarbonyl, methoxy, ethoxy, n- or i-propoxy, methylthio, ethylthio, n- or i-propylthio, methylsulphinyl, ethylsulphinyl, n- or i-propylsulphinyl, methylsulphonyl, ethylsulphonyl, n- or i-propylsulphonyl, represents in each case optionally fluorine-, chlorine- or bromine-substituted ethenyl, propenyl, butenyl, ethinyl, propinyl or butinyl, or represents in each case optionally cyano-, fluorine-, chlorine-, methyl- or ethyl-substituted cyclopropyl, cyclobutyl, cyclopentyl or cyclohexyl,
R⁴ represents amino, cyano, carbamoyl, thiocarbamoyl, formyl, fluorine, chlorine, bromine, represents in each case optionally cyano-, fluorine-, chlorine-, methoxy- or ethoxy-substituted methyl, ethyl, n- or i-propyl, acetyl, propionyl, n- or i-butyroyl, methoxy, ethoxy, n- or i-propoxy, methoxycarbonyl, ethoxycarbonyl, n- or i-propoxycarbonyl, methylthio, ethylthio, n- or i-propylthio, methylsulphinyl, ethylsulphinyl, n- or i-propylsulphinyl, methylsulphonyl, ethylsulphonyl, n- or i-propylsulphonyl, , methylamino, ethylamino, n- or i-propylamino, dimethylamino, diethylamino, acetylamino, propionylamino, n- or i-butyroylamino, methoxycarbonylamino, ethoxycarbonylamino, n- or i-propoxycarbonylamino, methylsulphonylamino, ethylsulphonylamino, n- or i-propylsulphonylamino, represents in each case optionally cyano-, fluorine-, chlorine- or bromine-substituted ethenyl, propenyl, butenyl, ethinyl, propinyl, butinyl, ethenylcarbonyl, propenylcarbonyl, butenylcarbonyl, ethinylcarbonyl, propinylcarbonyl or butinylcarbonyl, or represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy-, trifluoromethoxy-, methoxycarbonyl-, ethoxycarbonyl-, nor i-propoxycarbonyl-substituted phenyl, benzoyl or benzyl, and
R⁵ represents nitro, amino, cyano, carbamoyl, thiocarbamoyl, formyl, fluorine, chlorine, bromine, represents in each case optionally cyano-, fluorine-, chlorine-, methoxy- or ethoxy-substituted methyl, ethyl, n- or i-propyl, acetyl, propionyl, n- or i-butyroyl, methoxy, ethoxy, n- or i-propoxy, methoxycarbonyl, ethoxycarbonyl, n- or i-propoxycarbonyl, methylthio, ethylthio, n- or i-propylthio, methylsulphinyl, ethylsulphinyl, n- or i-propylsulphinyl, methylsulphonyl, ethylsulphonyl, nor i-propylsulphonyl, methylamino, ethylamino, n- or i-propylamino, dimethylamino, diethylamino, acetylamino, propionylamino, n- or i-butyroylamino, methoxycarbonylamino, ethoxycarbonylamino, n- or i-propoxycarbonylamino, methylsulphonylamino, ethylsulphonylamino, n- or i-propylsulphonylamino, represents in each case optionally cyano-, fluorine-, chlorine- or bromine-substituted ethenyl, propenyl, butenyl, ethinyl, propinyl, butinyl, ethenylcarbonyl, propenylcarbonyl, butenylcarbonyl, ethinylcarbonyl, propinylcarbonyl or butinylcarbonyl, or represents in each case optionally nitro-, cyano-, fluorine-, chlorine-, bromine-, methyl-, ethyl-, n- or i-propyl-, n-, i-, s-or t-butyl-, trifluoromethyl-, methoxy-, ethoxy-, n- or i-propoxy-, difluoromethoxy-, trifluoromethoxy-, methoxycarbonyl-, ethoxycarbonyl-, n- or i-propoxycarbonyl-substituted phenyl, benzoyl or benzyl.

4. Compounds according to any of Claims 1 to 3, **characterized in that**
A¹ represents methylene or dimethylene,
A² represents methylene or dimethylene,
A³ represents methylene or dimethylene,
R¹ represents hydrogen,
R² represents hydrogen, represents formyl or represents in each case optionally fluorine-, chlorine-, methoxy- or ethoxy-substituted acetyl, propionyl, n- or i-butyroyl, methoxycarbonyl, ethoxycarbonyl, n- or i-propoxycarbonyl, or
the grouping N(R¹R²) represents dimethylaminomethyleneamino,
R³ represents in each case optionally fluorine- or chlorine-substituted methyl, ethyl, n- or i-propyl,
R⁴ represents cyano, fluorine, chlorine, bromine, or represents in each case optionally fluorine- or chlorine-substituted methyl, ethyl, methoxy or ethoxy, and
R⁵ represents nitro, cyano, fluorine, chlorine, bromine, or represents in each case optionally fluorine- or chlorine-substituted methyl, ethyl, methoxy or ethoxy.

5. Compounds according to any of claims 1 to 4, **characterized in that**
Z represents 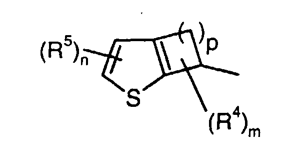 where
p represents 2, 3 or 4, and n, m, R⁴ and R⁵ are as defined in any of Claims 1 to 4.

6. Process for preparing substituted triazines according to any of Claims 1 to 5, **characterized in that** biguanides of the general formula (II) 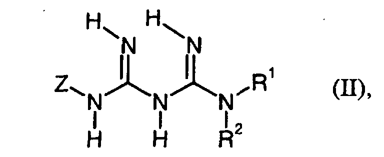 in which
R¹, R² and Z are as defined in any of Claims 1 to 5,
and/or acid adducts of compounds of the general formula (II)
are reacted with alkoxycarbonyl compounds of the general formula (III)
R³-CO-OR⁴ (III)
in which
R³ is as defined in any of Claims 1 to 4 and
R⁴ represents alkyl,
if appropriate in the presence of a reaction auxiliary and if appropriate in the presence of a diluent, and, if appropriate, further conversions within the scope of the definition of the substituents are carried out by customary methods on the resulting compounds of the general formula (I).

7. Compounds of the formula (II) 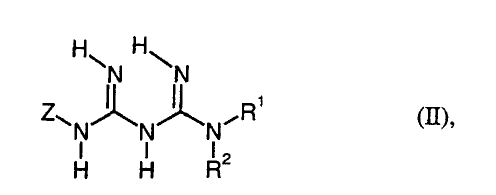 **characterized in that**
R¹, R² and Z are as defined in any of Claims 1 to 5,
and the acid adducts of the compounds of the general formula (II).

8. Process for preparing compounds according to Claim 7, **characterized in that** amino compounds of the general formula (IV)
Z-NH₂ (IV)
in which
Z is as defined in any of Claims 1 to 5,
and/or acid adducts of compounds of the general formula (IV)
are reacted with cyanoguanidine of the formula (V) 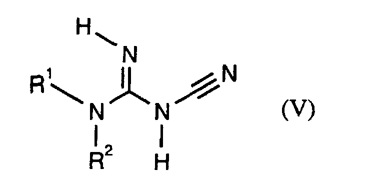 in which R¹ and R² are as defined in Claim 1,
if appropriate in the presence of a reaction auxiliary and if appropriate in the presence of a diluent at temperatures between 100°C and 200°C.

9. Method for controlling undesirable vegetation, **characterized in that** at least one compound according to any of Claims 1 to 5 is allowed to act on undesirable plants and/or their habitats.

10. Use of at least one compound according to any of Claims 1 to 5 for controlling undesirable plants.

11. Herbicidal composition, **characterized in that** it comprises a compound according to any of Claims 1 to 5 and customary extenders and/or surfactants.

## Revendications

1. Composés de formule générale (I) 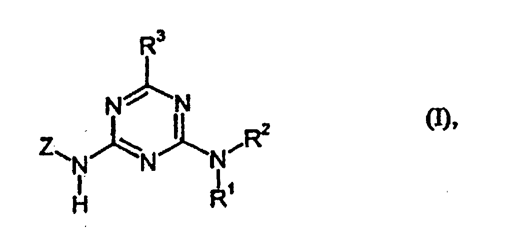 dans laquelle
R¹ représente l'hydrogène ou un groupe alkyle éventuellement substitué,
R² représente l'hydrogène, un groupe formyle ou un groupe alkyle, alkylcarbonyle, alkoxycarbonyle ou alkylaminocarbonyle éventuellement substitué dans chaque cas,
ou bien le groupement N(R¹R²) représente aussi un reste dialkylamino-alkylidène-amino,
R³ représente l'hydrogène, un halogène, un reste alkyle éventuellement substitué, un reste alkylcarbonyle, alkoxycarbonyle, alkoxy, alkylthio, alkylsulfinyle ou alkylsulfonyle dont chacun est éventuellement substitué, un reste alcényle ou alcynyle dont chacun est éventuellement substitué, ou un reste cycloalkyle éventuellement substitué, et
Z représente les groupements thiénocycloalkyl(alcényle) suivants : 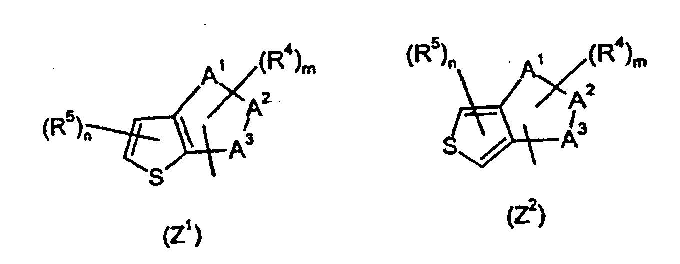
dans lesquels
m représente les nombres 0, 1, 2, 3 ou 4,
n représente les nombres 0, 1 ou 2,
A¹ représente O(oxygène), S(soufre), -CO-, -CS- ou un reste alcanediyle(alkylène),
A² représente O(oxygène), S(soufre), -CO-, -CS- ou un reste alcanediyle(alkylène),
A³ représente O(oxygène), S(soufre), -CO-, -CS- ou un reste alcanediyle(alkylène),
- sous réserve que l'un au moins des groupements A¹, A², A³ représente un reste alcanediyle et que deux groupes contigus ne représentent pas en même temps S ou O -
R⁴ est un reste amino, cyano, carbamoyle, thiocarbamoyle, formyle, un halogène, ou un reste alkyle, alkylcarbonyle, alkoxy, alkoxycarbonyle, alkylthio, alkylsulfinyle, alkylsulfonyle, alkylamino, dialkylamino, alkylcarbonylamino, alkoxycarbonylamino, alkylsulfonylamino, alcényle, alcynyle, alcénylcarbonyle, alcynylcarbonyle, aryle, arylcarbonyle ou arylalkyle, dont chacun est éventuellement substitué, et
R⁵ représente un reste nitro, amino, cyano, carbamoyle, thiocarbamoyle, formyle, un halogène, ou bien un reste alkyle, alkylcarbonyle, alkoxy, alkoxycarbonyle, alkylthio, alkylsulfinyle, alkylsulfonyle, alkylamino, dialkylamino, alkylcarbonylamino, alkoxycarbonylamino, alkylsulfonylamino, alcényle, alcynyle, alcénylcarbonyle, alcynylcarbonyle, aryle, arylcarbonyle ou arylalkyle, dont chacun est éventuellement substitué.

2. Composés suivant la revendication 1, **caractérisés en ce que**
m représente les nombres 0, 1 ou 2,
A¹ représente 0(oxygène), S(soufre), -CO-, -CS- ou un reste alcanediyle(alkylène) ayant 1 à 3 atomes de carbone,
A² représente O(oxygène), S(soufre), -CO-, -CS- ou un reste alcanediyle(alkylène) ayant 1 à 3 atomes de carbone,
A³ représente O(oxygène), S(soufre), -CO-, -CS- ou un reste alcanediyle(alkylène) ayant 1 à 3 atomes de carbone,
- sous réserve que l'un au moins des groupements A¹, A², A³ soit un reste alcanediyle ayant 1 à 3 atomes de carbone et que deux groupes contigus ne représentent pas en même temps S ou O -
R¹ représente l'hydrogène ou un reste alkyle ayant 1 à 6 atomes de carbone éventuellement substitué par un radical cyano, halogéno ou alkoxy en C₁ à C₄,
R² représente l'hydrogène, un reste formyle ou un reste alkyle, alkylcarbonyle, alkoxycarbonyle ou alkylaminocarbonyle ayant dans chaque cas 1 à 6 atomes de carbone dans les groupes alkyle et chacun portant éventuellement un substituant cyano, halogéno ou alkoxy en C₁ à C₄, ou bien
le groupement N(R¹R²) représente un reste dialkylamino-alkylidène-amino ayant jusqu'à 4 atomes de carbone dans chacun des groupes alkyle ou alkylidène,
R³ représente l'hydrogène, un halogène, un reste alkyle ayant 1 à 6 atomes de carbone portant éventuellement un substituant cyano, halogéno, hydroxy, alkoxy en C₁ à C₄ ou alkylthio en C₁ à C₄, un reste alkylcarbonyle, alkoxycarbonyle, alkoxy, alkylthio, alkylsulfinyle ou alkylsulfonyle ayant chacun 1 à 6 atomes de carbone dans les groupes alkyle et portant chacun, le cas échéant, un substituant cyano, halogéno ou alkoxy en C₁ à C₄, un reste alcényle ou alcynyle ayant chacun 2 à 6 atomes de carbone et portant chacun éventuellement un substituant halogéno ou un reste cycloalkyle de 3 à 6 atomes de carbone portant éventuellement un substituant cyano, halogéno ou alkyle en C₁ à C₄,
R⁴ est un reste amino, cyano, carbamoyle, thiocarbamoyle, formyle, un halogène, un reste alkyle, alkylcarbonyle, alkoxy, alkoxycarbonyle, alkylthio, alkylsulfinyle, alkylsulfonyle, alkylamino, dialkylamino, alkylcarbonylamino, alkoxycarbonylamino ou alkylsulfonylamino ayant chacun 1 à 6 atomes de carbone dans les groupes alkyle et portant chacun, le cas échéant, un substituant cyano, halogéno ou alkoxy en C₁ à C₄, un reste alcényle, alcynyle, alcénylcarbonyle ou alcynylcarbonyle ayant chacun 2 à 6 atomes de carbone dans les groupes alcényle ou alcynyle et portant chacun, le cas échéant, un substituant cyano ou halogéno, ou bien un reste aryle, arylcarbonyle ou arylalkyle ayant chacun 6 ou 10 atomes de carbone dans le groupe aryle et, le cas échéant, 1 à 4 atomes de carbone dans la partie alkyle et portant chacun, le cas échéant, un substituant nitro, cyano, halogéno, alkyle en C₁ à C₄, halogénalkyle en c₁ à C₄, alkoxy en C₁ à C₄, halogénalkoxy en C₁ à C₄ ou (alkoxy en C₁ à C₄)carbonyle, et
R⁵ est un reste nitro, amino, cyano, carbamoyle, thiocarbamoyle, formyle, un halogène, un reste alkyle, alkylcarbonyle, alkoxy, alkoxycarbonyle, alkylthio, alkylsulfinyle, alkylsulfonyle, alkylamino, dialkylamino, alkylcarbonylamino, alkoxycarbonylamino ou alkylsulfonylamino ayant chacun 1 à 6 atomes de carbone dans les groupes alkyle et portant chacun, le cas échéant, un substituant cyano, halogéno ou alkoxy en C₁ à C₄, un reste alcényle, alcynyle, alcénylcarbonyle ou alcynylcarbonyle ayant chacun 2 à 6 atomes de carbone dans les groupes alcényle ou alcynyle et portant chacun, le cas échéant, un substituant cyano ou halogéno, ou bien un reste aryle, arylcarbonyle ou arylalkyle ayant dans chaque cas 6 ou 10 atomes de carbone dans le groupe aryle et, le cas échéant, 1 à 4 atomes de carbone dans la partie alkyle et portant chacun éventuellement un substituant nitro, cyano, halogéno, alkyle en C₁ à C₄, halogénalkyle en C₁ à C₄, alkoxy en C₁ à C₄, halogénalkoxy en C₁ à C₄ ou (alkoxy en C₁ à C₄)carbonyle.

3. Composés suivant la revendication 1 ou 2, **caractérisés en ce que**
A¹ représente O(oxygène), S(soufre), -CO-, -CS-, un reste méthylène, diméthylène ou triméthylène,
A² représente O(oxygène), S(soufre), -CO-, -CS-, un reste méthylène, diméthylène ou triméthylène,
A³ représente O(oxygène), S(soufre), -CO-, -CS-, un reste méthylène, diméthylène ou triméthylène,
- sous réserve qu'au moins l'un des groupements A¹, A², A³ représente un reste méthylène, diméthylène ou triméthylène et que deux groupes contigus ne représentent pas en même. temps S ou O -
R¹ est l'hydrogène ou un reste méthyle, éthyle, n-propyle
ou isopropyle portant chacun, le cas échéant, un substituant cyano, fluoro, chloro, méthyle ou éthoxy,
R² représente l'hydrogène, un reste formyle ou un reste méthyle, éthyle, n-propyle, isopropyle, acétyle, propionyle, n-butyroyle, isobutyroyle, méthoxycarbonyle, éthoxycarbonyle, n-propoxycarbonyle, isopropoxycarbonyle, méthylaminocarbonyle, éthylaminocarbonyle, n-propylaminocarbonyle ou isopropylaminocarbonyle portant chacun, le cas échéant, un substituant cyano, fluoro, chloro, méthoxy ou éthoxy, ou bien
le groupement N(R¹R²) représente un reste diméthylaminométhylène-amino ou diéthylaminométhylène-amino,
R³ représente l'hydrogène, le fluor, le chlore, le brome, un reste méthyle, éthyle, n-propyle, isopropyle, n-butyle, isobutyle, sec.-butyle portant chacun, le cas échéant, un substituant cyano, fluoro, chloro, bromo, hydroxy, méthoxy, éthoxy, n-propoxy, isopropoxy, méthylthio, éthylthio, n-propylthio ou isopropylthio, un reste acétyle, propionyle, n-butyroyle, isobutyroyle, méthoxycarbonyle, éthoxycarbonyle, n-propoxycarbonyle, isopropoxycarbonyle, méthoxy, éthoxy, n-propoxy, isopropoxy, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle ou isopropylsulfonyle portant chacun, le cas échéant, un substituant cyano, fluoro, chloro, méthoxy ou éthoxy, un reste éthényle, propényle, butényle, éthynyle, propynyle ou butynyle portant chacun, le cas échéant, un substituant fluoro, chloro ou bromo, ou bien un reste cyclopropyle, cyclobutyle, cyclopentyle ou cyclohexyle portant chacun, le cas échéant, un substituant cyano, fluoro, chloro, méthyle ou éthyle,
R⁴ est un reste amino, cyano, carbamoyle, thiocarbamoyle, formyle, le fluor, le chlore, le brome, un reste méthyle, éthyle, n-propyle, isopropyle, acétyle, propionyle, n-butyroyle, isobutyroyle, méthoxy, éthoxy, n-propoxy, isopropoxy, méthoxycarbonyle, éthoxycarbonyle, n-propoxycarbonyle, isopropoxycarbonyle, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle, isopropylsulfonyle, méthylamino, éthylamino, n-propylamino, isopropylamino, diméthylamino, diéthylamino, acétylamino, propionylamino, n-butyroylamino, isobutyroylamino, méthoxycarbonylamino, éthoxycarbonylamino, n-propoxycarbonylamino, isopropoxycarbonylamino, méthylsulfonylamino, éthylsulfonylamino, n-propylsulfonylamino, isopropylsulfonylamino portant chacun, le cas échéant, un substituant cyano, fluoro, chloro, méthoxy ou éthoxy, un reste éthényle, propényle, butényle, éthynyle, propynyle, butynyle, éthénylcarbonyle, propénylcarbonyle, buténylcarbonyle, éthynylcarbonyle, propynylcarbonyle ou butynylcarbonyle portant chacun, le cas échéant, un substituant cyano, fluoro, chloro ou bromo, ou bien reste phényle, benzoyle ou benzyle portant chacun, le cas échéant, un substituant nitro, cyano, fluoro, chloro, bromo, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy, trifluorométhoxy, méthoxycarbonyle, éthoxycarbonyle, n-propoxycarbonyle ou isopropoxycarbonyle, et
R⁵ est un reste nitro, amino, cyano, carbamoyle, thiocarbamoyle, formyle, le fluor, le chlore, le brome, un reste méthyle, éthyle, n-propyle, isopropyle, acétyle, propionyle, n-butyroyle, isobutyroyle, méthoxy, éthoxy, n-propoxy, isopropoxy, méthoxycarbonyle, éthoxycarbonyle, n-propoxycarbonyle, isopropoxycarbonyle, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle, isopropylsulfonyle, méthylamino, éthylamino, n-propylamino, isopropylamino, diméthylamino, diéthylamino, acétylamino, propionylamino, n-butyroylamino, isobutyroylamino, méthoxycarbonylamino, éthoxycarbonylamino, n-propoxycarbonylamino, isopropoxycarbonylamino, méthylsulfonylamino, éthylsulfonylamino, n-propylsulfonylamino, isopropylsulfonylamino portant chacun, le cas échéant, un substituant cyano, fluoro, chloro, méthoxy ou éthoxy, un reste éthényle, propényle, butényle, éthynyle, propynyle, butynyle, éthénylcarbonyle, propénylcarbonyle, buténylcarbonyle, éthynylcarbonyle, propynylcarbonyle ou butynylcarbonyle portant chacun, le cas échéant, un substituant cyano, fluoro, chloro ou bromo, ou bien un reste phényle, benzoyle ou benzyle portant chacun, le cas échéant, un substituant nitro, cyano, fluoro, chloro, bromo, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy, trifluorométhoxy, méthoxycarbonyle, éthoxycarbonyle, n-propoxycarbonyle ou isopropoxycarbonyle.

4. Composés suivant l'une des revendications 1 à 3, **caractérisés en ce que**
A¹ est un reste méthylène ou diméthylène,
A² est un reste méthylène ou diméthylène,
A³ est un reste méthylène ou diméthylène,
R¹ est l'hydrogène,
R² est l'hydrogène, un reste formyle ou un reste acétyle, propionyle, n-butyroyle, isobutyroyle, méthoxycarbonyle, éthoxycarbonyle, n-propoxycarbonyle ou isopropoxycarbonyle portant chacun, le cas échéant, un substituant fluoro, chloro, méthoxy ou éthoxy, ou bien le groupement N(R¹R²) représente un reste diméthylaminométhylène-amino,
R³ est un reste méthyle, éthyle, n-propyle ou isopropyle portant chacun, le cas échéant, un substituant fluoro
ou chloro,
R⁴ est un reste cyano, le fluor, le chlore, le brome ou un reste méthyle, éthyle, méthoxy ou éthoxy portant chacun, le cas échéant, un substituant fluoro ou chloro, et
R⁵ est un reste nitro, cyano, le fluor, le chlore, le brome, ou un reste méthyle, éthyle, méthoxy ou éthoxy portant chacun, le cas échéant, un substituant fluoro
ou chloro.

5. Composés suivant l'une des revendications 1 à 4, **caractérisés en ce que**
Z est un reste de formule 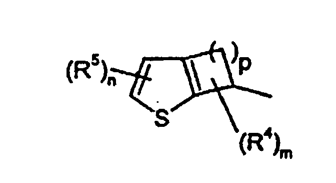
dans laquelle
p a la valeur 2, 3 ou 4 et n, m,R⁴ et R⁵ ont la définition indiquée dans l'une des revendications 1 à 4.

6. Procédé de production de triazines substituées suivant l'une des revendications 1 à 5, **caractérisé en ce qu'**on fait réagir des biguanides de formule générale (II) 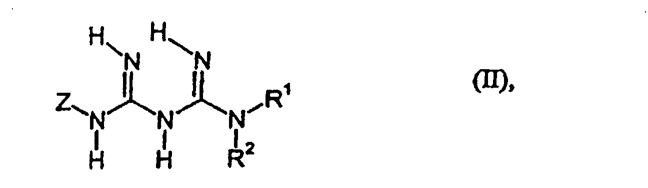 dans laquelle
R¹, R² et Z ont la définition indiquée dans l'une des revendications 1 à 5,
et/ou des produits d'addition d'acides de composés de formule générale (II)
avec des composés alkoxycarbonyliques de formule générale (III)
R³-CO-OR⁴ (III)
dans laquelle
R³ a la définition indiquée dans l'une des revendications 1 à 4 et
R⁴ est un reste alkyle,
le cas échéant en présence d'un auxiliaire de réaction et en présence éventuelle d'un diluant et on effectue, le cas échéant, sur les composés obtenus de formule générale (I), dans le cadre de la définition des substituants, d'autres transformations selon des procédés classiques.

7. Composés de formule (II) 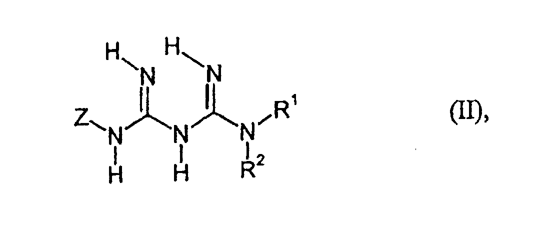 **caractérisés en ce que**
R¹, R² et Z ont la définition indiquée dans l'une des revendications 1 à 5,
ainsi que les produits d'addition d'acides des composés de formule générale (II).

8. Procédé de production de composés suivant la revendication 7, **caractérisé en ce qu'**on fait réagir des composés aminés de formule générale (IV)
Z-NH₂ (IV)
dans laquelle
Z a la définition indiquée dans l'une des revendications 1 à 5,
et/ou des produits d'addition d'acides de composés de formule (IV)
avec la cyanoguanidine de formule (V) 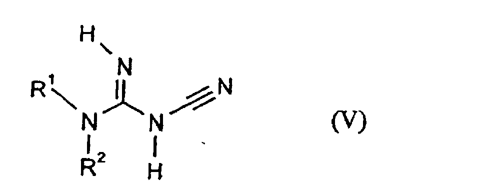 dans laquelle
R¹ et R² ont la définition indiquée dans la revendication 1,
le cas échéant en présence d'un auxiliaire de réaction et en présence éventuelle d'un diluant, à des températures comprises entre 100°C et 200°C.

9. Procédé pour combattre une végétation indésirable, **caractérisé en ce qu'**on fait agir au moins un composé suivant l'une des revendications 1 à 5 sur des plantes indésirables et/ou sur leur milieu.

10. Utilisation d'au moins un composé suivant l'une des revendications 1 à 5 pour combattre des plantes indésirables.

11. Composition herbicide, **caractérisée en ce qu'**elle contient un composé suivant l'une des revendications 1 à 5 et des diluants et/ou agents tensio-actifs classiques.
